# EUROPEAN PATENT APPLICATION

(11) **EP 4 279 067 A1**
(43) Date of publication of application: **22.11.2023**
(21) Application number: 22739023.4
(22) Date of filing: 12.01.2022
(51) Int. Cl.: A61K 9/127, A61K 9/06, A61K 45/00, A61K 47/36, A61K 47/42, B82Y 5/00, B82Y 30/00, B82Y 40/00

(54) **NANOCOMPLEX, PREPARATION METHOD THEREFOR, AND USE THEREOF**

(30) Priority: 13.01.2021 CN 202110040371
(71) Applicant: Shanghai Jiaotong University School of Medicine, Shanghai 200025 (CN)
(72) Inventor: GAO, Xiaoling, Chongqing Road, Huangpu District Shanghai 200025 (CN); HUANG, Jialin, Chongqing Road, Huangpu District Shanghai 200025 (CN); JIANG, Gan, Chongqing Road, Huangpu District Shanghai 200025 (CN); SONG, Qingxiang, Chongqing Road, Huangpu District Shanghai 200025 (CN); CHEN, Hongzhuan, Chongqing Road, Huangpu District Shanghai 200025 (CN)
(74) Representative: dompatent von Kreisler Selting Werner - Partnerschaft von Patent- und Rechtsanwälten mbB
(86) International application number: PCT/CN2022/071526
(87) International publication number: WO 2022/152145

(57) **Abstract**

Disclosed is a nanocomplex, a preparation method therefor, and a use thereof. The nanocomplex comprises 0-60% a protein drug, 0.03-15% hyaluronic acid, 0.8-20% protamine, 35-95% lipid components, and 2.5-40% a apolipoprotein and/or a mimetic peptide thereof; the lipid components comprise an electrically neutral lipid and an anionic lipid; and the total amount of hyaluronic acid and protamine is 0.03-15%. The nanocomplex of the present invention provides a general-type carrier for protein drugs having different physicochemical properties (such as molecular weights of 10-255 KDa and PIs of 4-11), implements highly efficient intracellular, in vivo, and even in-brain delivery, utilized technology is universal in nature, and said invention can effectively solve the current problem of insufficient in vivo and in vitro transport of protein drugs (comprising a protein drug exceeding the molecular weight and PI of an embodiment).

## Description

The present application claims the priority of Chinese patent application 2021100403712 with the filing date of 2021/1/13. The contents of the above Chinese patent applications are incorporated herein by reference in its entirety.

### TECHNICAL FIELD

The present invention relates to the field of biotechnology as well as to the field of chemical pharmaceuticals, in particular to a nanocomplex for protein drug delivery, a preparation method therefor and a use thereof as a carrier for protein drug delivery.

### BACKGROUND

As an executor of cellular functions, proteins play an important role in pathophysiological processes, such as cell metabolism, gene regulation, signal transduction and immune response. Protein drug therapy involves the introduction of normal protein drugs into specific tissues or cells of a patient to correct dysfunctional or missing proteins for therapeutic purposes. Compared with traditional small-molecule drugs, the protein drugs have the advantages of high biological activity, strong specificity and clear biological function. Compared with gene therapy, the protein drugs are more rapid and safe, can directly exert their biological functions at specific sites, and do not involve changes in gene sequences. In recent years, the protein drugs have become the focus of most attention in the field of medicine, and have occupied the top 10 best-selling drugs in the world for many years. However, due to the lack of effective protein drug delivery means, the targets of marketed protein drugs are mainly limited to the extracellular molecules, for example, antibodies, coming into play, which greatly limits the large-scale development and application of protein drugs.

Currently, there are two main categories of means of protein delivery. The first type of method provides a molecule which directly covalently links or fuses a protein of interest to facilitate endocytosis, e.g., for linking a transmembrane peptide, a targeting molecule, etc. that facilitate uptake of the protein by target cells. Although this method can efficiently deliver proteins into cells, the proteins that enter cells often lack the ability to escape from the endosome of the cell, resulting in degradation of the protein by the endosome when the function of the protein has not been performed. While this method involves a complex synthesis and purification process, the structure and function of the protein are likely to be destroyed in this process. The second type of method is the use of carrier materials to deliver proteins through specific recognition between molecules or cationic carrier materials. Protein delivery carriers are broadly divided into two major classes depending on the manner of binding between the carrier material and the protein. The first class of carrier materials generally bind proteins through some specific recognition existing between molecules (such as recognition between biotin and avidin, his-tag and metal ions, transcription factors and promoters) or chemical reactions (such as amino and azide, tetrazine (Tz) and TCO, etc.). These carrier materials have a strong binding ability to proteins. However, the preparation process involves a complex protein modification process and carrier material synthesis modification process. Meanwhile, the specific molecular markers of proteins and its covalent binding to support materials may destroy the structure and function of proteins. Thus, most current research has been focused on the second type of carrier materials, namely cationic carrier materials, which bind directly to target proteins via electrostatic or hydrophobic interactions. Since protein molecules have a complex three-dimensional structure, and their surface charges and hydrophilic-hydrophobic groups are not uniformly distributed. Most of the protein surfaces have partial hydrophobic and negative regions, and thus can be directly bound and delivered via the electrostatic or hydrophobic interactions using the cationic carriers. For example, Cheng's research group directly constructed a library of cationic materials by grafting different small fluorine-containing compounds onto polyethyleneimine to obtain a library of fluorine-containing polymer materials, further screened the protein delivery properties of the polymer materials in the library, and successfully obtained two high-performance fluorine-containing high molecular materials. These fluorine-containing high molecules can efficiently deliver a variety of protein molecules including bovine serum albumin, β-galactosidase, saporin, etc. into different cells and maintain the biological activity of these proteins or small peptides. However, considering the toxicity of cationic materials in vivo, such carriers are mostly limited to protein transfection in vitro. In the case of positively charged proteins, researchers can modify proteins by super-negative charging to make the whole negatively charged, and then carry it with cationic carrier materials through electrostatic interaction. For example, the Rotello's research group inserted small polyglutamic acid peptides of different lengths into the N-terminal of Cas9 protein, so that the positively charged Cas9 protein has sufficient negative charge regions, and co-incubated the protein with sgRNA to form a negative complex, which co-incubated with arginine-modified gold nanoparticles by electrostatic interaction to form a nano-complex by self-assembly. It was found that the complex can be efficiently taken up by HeLa cells by a cholesterol-dependent membrane fusion mode, achieving efficient editing of two genes, AAVS1 and PTEN. However, although the delivery of proteins with different charge characteristics can be achieved by using super-negatively charged modifications, more complex biological or chemical synthesis of the protein is also required, and the structure and function of the protein may be destroyed during the modification process. Thus, there is a lack of a convenient and versatile method to achieve efficient protein drug carrying and in vivo delivery.

Recombinant lipoprotein is a kind of nanoparticle which mimics the natural nanostructure-lipoprotein, and has been developed as a widely used nano-drug carrier, which has the following advantages. (1) It has good biocompatibility, can be completely metabolized by the body and has no immunogenicity. (2) Long circulation time in vivo. (3) Multi-mode drug carrying. (4) Targeting properties in vivo. Recombinant lipoprotein nanocarriers have been successfully applied to the effective delivery of partially hydrophobic drugs, amphipathic drugs and small amounts of hydrophilic drugs such as polypeptides and nucleic acid drugs. In this study, we established targeted intracerebral delivery of neuroprotective peptides to the lesion site of Alzheimer's disease with β-amyloid aggregation through the establishment of α-helical peptide fusion technology (ACS Nano, 2015). The development of hydrophobic co-precipitation technology was proposed to introduce a calcium phosphate inner core into a recombinant lipoprotein shell to achieve efficient nucleic acid drug carrying and targeted delivery of glioma cells (Nat Commun, 2017 & 2020; Adv Sci, 2020). However, since the activity of a protein drug is highly dependent on its complex high-order structure, the existing hydrophobic co-precipitation "hard core" type drug encapsulation method can only achieve a physical barrier between the protein and the external environment, and cannot avoid the structural damage and activity loss of the inner core protein under precipitation conditions. Therefore, there is an urgent need to develop a carrying method suitable for protein drugs in order to achieve highly efficient targeted delivery thereof.

In previous studies, Professor Leaf Huang's group invented a so-called cationic liposome-polycation-hyaluronic acid nanocomplex for targeted delivery of gene drugs to tumor cells by linking targeting groups at the end of a PEG phospholipid molecule for specific recognition of tumors. Although hyaluronic acid and protamine complexes have been used for nucleic acid drug delivery, they can be used for in vivo delivery by coating liposomes containing cationic lipids and pegylated phospholipids, as an outer shell. However, these systems cannot be used directly for protein drug delivery, and there is a risk of non-specific cytotoxicity, allergic reactions, etc.

### SUMMARY OF THE INVENTION

The technical problem to be solved by the present invention is to overcome the lack of a suitable method for carrying protein drugs in the prior art, so as to achieve highly efficient intracellular and in vivo targeted delivery thereof, and to provide a nanocomplex, a preparation method therefor, and a use thereof.

The present invention solves the above technical problem by the following technical solution.

A first object of the present invention is to provide a nanocomplex comprising 0-60% of protein drug, 0.03-15% of hyaluronic acid, 0.1-20% of protamine, 35-95% of lipid component, and 2.5-40% of "apolipoprotein and/or a mimetic peptide thereof';
the lipid component comprises an electrically neutral lipid and an anionic lipid; the total amount of the hyaluronic acid and the protamine used is 0.03-15%;
the above percentages are the respective mass percentages of the components relative to the nanocomplex.

In the present invention, when the protein drug content is 0%, the nanocomplex is an empty carrier not carried with the protein drug. When the protein drug content is not zero, the nanocomplex is a protein drug-carried nanocomplex.

In the present invention, it is preferable that the cationic lipid DOTAP is not included in the nanocomplex.

In the present invention, the protein drug may be a protein drug conventional in the art.

In the present invention, the molecular weight of the protein drug may be 10-255 kDa, such as 10-16 kDa, 15-40 kDa, 30-50 kDa, 60-80 kDa 140-180 kDa, 200-255 kDa or 210-255 kDa, preferably 10-14 kDa, 20-33 kDa, 35-45 kDa, 60-80 kDa, 150-170 kDa, 220-250 kDa, 220-250 kDa, and further, such as 12.4 kDa, 26 kDa, 40 kDa, 69.3 kDa, 160 kDa or 240 kDa.

In the present invention, the isoelectric point of the protein drug may be 4-11, such as 4-5.3, 3.7-5.7, 4.4-6.4, 6-8.5, 7-9, 8.3-10.3 or 9.3-11, preferably 4-4.8, 4.2-5.5, 4.9-5.9, 6.5-8, 7.5-8.5, 8.8-9.8 or 9.8-10.8, and further, such as 4.3, 4.7, 5.4, 7.2, 8, 9.3 or 10.3.

In the present invention, the isoelectric point of the protein drug may also be 4-6, 6-8 or 8-10.5.

In a preferred embodiment of the invention, the following protein drugs may be used.

| Protein | Isoelectric point | Molecular weight (kDa) |
|---|---|---|
| Phycoerythrin PE | 4.3 | 240 |
| Bovine serum albumin BSA | 4.7 | 69.3 |
| Catalase (CAT) | 5.4 | 250 |
| Horseradish peroxidase (HRP) | 7.2 | 40 |
| IgG antibody | 8 | 160 |
| Nerve growth factor (β-NGF) | 9.3 | 26 |
| Cytochrome C (CC) | 10.3 | 12.4 |

In the present invention, the electrically neutral lipid generally refers to a lipid that is in a positive and negative ion balance in a solution, shows no charged property to the outside of the whole solution, and preferably includes one or more of an amphipathic lipid, a nonionic lipid, cholesterol and derivatives thereof. More preferably, one or more of phosphatidyl choline, phosphatidyl ethanolamine, phosphatidylglycerol and sphingomyelin are included.

Herein, the phosphatidylcholine is preferably one or more of dimyristoyl phosphatidylcholine (DMPC), dipalmitoyl phosphatidylcholine (DPPC), distearoyl phosphatidylcholine (DSPC), dioleoyl phosphatidylcholine (DOPC), dilaroyl phosphatidylcholine (DLPC), dierucoyl phosphatidylcholine (DEPC), 1-palmitoyl-2-oleoyl phosphatidylcholine (POPC), egg yolk lecithin, soybean phospholipid, hydrogenated soybean phosphatidylcholine (HSPC) and derivatives thereof, more preferably, one or more of dimyristoyl phosphatidylcholine (DMPC), egg yolk lecithin and hydrogenated soybean phosphatidylcholine HSPC, such as DMPC and egg yolk lecithin, or DMPC and hydrogenated soybean phosphatidylcholine.

The phosphatidyl ethanolamine is preferably one or more of dimyristoyl phosphatidyl ethanolamine (DMPE), distearoyl phosphatidyl ethanolamine (DSPE), dipalmitoyl phosphatidyl ethanolamine (DPPE), dioleoyl phosphatidyl ethanolamine (DOPE), distearoyl phosphatidyl ethanolamine-polyethylene glycol 2000, distearoyl phosphatidyl ethanolamine-polyethylene glycol 5000, dipalmitoyl phosphatidyl ethanolamine-polyethylene glycol 2000, dipalmitoyl phosphatidyl ethanolamine-polyethylene glycol 5000, and derivatives thereof.

The phosphatidylglycerol is preferably one or more of dimyristoyl phosphatidylglycerol (DMPG), distearoyl phosphatidylglycerol (DSPG), dipalmitoyl phosphatidylglycerol (DPPG), dioleoyl phosphatidylglycerol (DOPG), 1-palmitoyl-2-oleoyl phosphatidylglycerol (POPG-Na), egg yolk phosphatidylglycerol (EPG) and derivatives thereof.

Preferably, when the electrically neutral lipid is a mixture of dimyristoyl phosphatidylcholine (DMPC) and egg yolk lecithin, the mass ratio of dimyristoyl phosphatidylcholine (DMPC) to egg yolk lecithin may be 20:(8-12), for example 20:10.94.

In the present invention, the anionic lipid generally refers to a lipid, preferably an anionic phospholipid, having a negative charge greater than a positive charge in the solution and exhibiting a negative charge property to the outside of the whole solution. Preferably, the anionic phospholipid comprises one or more of phosphatidic acid, phosphatidylinositol, phosphatidylserine, cardiolipin, lysophospholipin and ganglioside, more preferably phosphatidic acid and/or ganglioside.

The phosphatidic acid is preferably one or more of dimyristyl phosphatidic acid (DMPA), distearoyl phosphatidic acid (DSPA), dipalmitoyl phosphatidic acid (DPPA), dioleoyl phosphatidic acid (DOPA) and derivatives thereof, more preferably, dimyristyl phosphatidic acid (DMPA), distearoyl phosphatidic acid (DSPA), dipalmitoyl phosphatidic acid (DPPA) or dioleoyl phosphatidic acid (DOPA).

Preferably, the phosphatidylserine is dioleoyl phosphatidylserine (DOPS) and/or dipalmitoyl phosphatidylserine (DPPS).

The lysophospholipid is preferably one or more of stearoyl lysolecithin S-lysoPC, myristoyl lysolecithin M-LysoPC, palmitoyl lysolecithin P-LysoPC and derivatives thereof.

The ganglioside is preferably monosialotetrahexosylganglioside (GM1).

Preferably, when the anionic phospholipid is a mixture of phosphatidic acid and ganglioside, the mass ratio of ganglioside to phosphatidic acid may be 30.07:(3-7), e.g., 30.07:5.

In the present invention, the "apolipoprotein and/or the mimetic peptide thereof" may be one or more of ApoE and a mimetic peptid thereof, ApoA-I, ApoA-II, ApoA-IV and a mimetic peptide thereof, ApoC-I, ApoC-II, ApoC-III and a mimetic peptide thereof, ApoB and a mimetic peptide thereof, ApoJ and a mimetic peptide thereof, e.g., ApoE or a mimetic peptide of ApoA-I.

In the present invention, the protein drug may be used in an amount of 1-43%, for example, 1.5%, 1.78%, 2.2%, 3%, 4%, 4.8%, 5%, 6%, 8%, 8.5%, 9%, 10%, 11%, 12.06%, 15%, 16%, 16.5%, 17%, 17.73%, 19%, 20%, 22%, 28.08%, 28.68%, 29.31%, 30%, 31.5%, 32%, 30.9%, 33%, 33.07%, 34%, 34.5% or 41%.

In the present invention, the hyaluronic acid may be used in an amount of 0.03%-5%, for example, 0.04%, 0.05%, 0.06%, 0.08%, 0.09%, 0.1%, 0.13%, 0.14%, 0.15%, 0.2%, 0.24%, 0.25%, 0.3%, 0.33%, 0.35%, 0.41%, 0.43%, 0.44%, 0.45%, 0.47%, 0.5%, 0.6%, 0.78%, 0.8%, 0.9%, 1.53%, 2.1%, 0.2%, 2.1%, 2.5% or 3%.

In the present invention, the protamine may be used in an amount of 0.2%-16%, for example, 0.1%, 0.2%, 0.3%, 0.35%, 0.8%, 0.83%, 1%, 1.3%, 1.5%, 1.6%, 1.7%, 2%, 2.34%, 2.39%, 2.44%, 3%, 3.26%, 3.3%, 3.5%, 3.7%, 4%, 4.3%, 4.5%, 4.85%, 5%, 5.1%, 5.5%, 5.53%, 6%, 6.5%, 7%, 13% or 14%.

In the present invention, the apolipoprotein and/or a mimetic peptide thereof may be used in an amount of 2.85%-25%, for example, 2.92%, 3%, 3.5%, 3.82%, 3.94%, 4%, 4.1%, 4.12%, 4.43%, 4.5%, 4.65%, 4.68%, 4.78%, 4.83%, 4.89%, 5%, 5.4%, 5.8%, 6%, 6.35%, 6.52%, 7.21%, 10.35%, 16.5%, 16.37%, 19.29% or 20%.

In the present invention, the lipid component is preferably used in an amount of 35%-90%.

In the present invention, the anionic lipid may be used in an amount of 14%-40%, for example 16%, 17.64%, 22%, 24%, 24.04%, 24.78%, 24.8%, 25%, 26%, 25.66%, 26.1%, 27%, 27.22%, 27.5%, 29.79%, 29%, 30%, 30.5%, 30.66%, 31%, 31.08%, 31.11%, 32%, 31.5%, 32%, 32.5%, 33%, 33.06%, 33.12%, 33.6%, 34%, 34.44%, 35%, 35.07%, 36%, 37.22%, 37.72%, 37.8%, 38% or 39%.

In the present invention, the electrically neutral lipid may be used in an amount of 22%-60%, for example, 23.5%, 24.36%, 30%, 30.94%, 32%, 33%, 34.2%, 34.22%, 35%, 36.09%, 37%, 37.44%, 37.5%, 38.25%, 39.08%, 39.91%, 41%, 41.21%, 42%, 42.34%, 42.92%, 43.5%, 44%, 45%, 45.61%, 46%, 46.4%, 47%, 47.56%, 48.43%, 50%, 52.17%, 52.2%, 53%, 54%, 54.5%, 55.32%, 56% or 57%.

In the present invention, the total amount of the hyaluronic acid and the protamine may be 0.2%-14%, for example, 0.3%, 0.5%, 0.64%, 0.8%, 0.85%, 0.97%, 1%, 1.08%, 1.15%, 1.2%, 1.5%, 1.63%, 1.71%, 1.79%, 2%, 2.15%, 2.35%, 2.58%, 2.63%, 2.68%, 3.2%, 3.5%, 4%, 4.17%, 5%, 5.08%, 5.5%, 5.6%, 5.96%, 6.38%, 6.5%, 7%, 8%, or 8.6%, 13.06%, or 13.5%.

In the present invention, the particle size of the nanocomplex may be 10-1000 nm, preferably 10-100 nm, for example 12-95 nm, further for example, 20.30±5.89 nm, 23.79±7.91 nm, 25 nm, 26.52±4.31 nm, 27.31±10.84 nm, 27.38±7.83 nm, 27.55±6.99 nm, 37.98±14.29 nm, 28.96±8.74 nm, 31.11±3.44 nm, 36.30±6.41 nm, 37.22±7.28 nm, 37.55±13.73 nm, 37.63±4.20 nm, 37.68±2.20 nm, 38 nm, 39.12±4.84 nm, 40.55±7.66 nm, 55 nm, 55.75±7.69 nm, 57 nm, 60 nm, 63.62±1.97 nm, 70 nm, 74.20±14.23 nm, or 75.29 ± 14.53 nm.

In the present invention, the nanocomplex may have a Zeta potential of -70 to -15 mV, such as -65, -64.87±3.30, -63.7±2.66, -58.87±4.90, -57.33±2.31, -56.33±3.26, -55.20±10.74, - 52.07±2.15, -50.10±3.18, -48.87±1.95, -45.20±2.15, -44.87±0.45 mV, -43.93±14.03, - 43.87±9.68, -43.20±2.75 mV, -40.23±6.92, -38.27±13.10, -36.83±2.71, -31.57±4.67, -30, -25, -20, -21.03±2.47, or -19.43±1.96 mV

As a preferred embodiment, when the isoelectric point of the protein drug is 4-5.7 and the molecular weight is 60-80 kDa, the nanocomplex comprises 0-30% of protein drug, 0.15-2.1% of hyaluronic acid, 1-6.5% of protamine, 4.65-7% of "apolipoprotein and/or a mimetic peptide thereof", 30-53.5% of electrically neutral phospholipid and 23.5-38.5% of anionic phospholipid; wherein the total amount of the hyaluronic acid and the protamine used is 1-9%.

The isoelectric point of the protein drug may be 4.2-5.5, for example, 4.7.

The molecular weight of the protein drug may be 60-80 kDa, for example, 69.3 kDa.

Herein the protein drug may be bovine serum albumin.

Herein the protein drug may be used in an amount of 0%, 9%, 16%, 22%, 28.08%, 28.68%, 29.31% or 30%.

The hyaluronic acid may be used in an amount of 0.15%, 0.24%, 0.33%, 0.35%, 0.47% or 2.1%.

The protamine may be used in an amount of 1%, 2%, 2.34%, 2.39%, 2.44%, 3.26%, 3.7% or 6.5%.

Herein the "apolipoprotein and/or mimetic peptide thereof" may be used in an amount of 4.65%, 4.68%, 4.78%, 4.83%, 4.89%, 5.4%, 6.35% or 6.52%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, for example, ApoE.

The electrically neutral phospholipid may be one or more of dimyristoyl phosphatidylcholine DMPC, egg yolk lecithin and hydrogenated soy lecithin HSPC, such as DMPC and egg yolk lecithin, or DMPC and hydrogenated soy lecithin.

When the electrically neutral phospholipid is a mixture of dimyristoyl phosphatidylcholine DMPC and egg yolk lecithin, the mass ratio of dimyristoyl phosphatidylcholine DMPC to egg yolk lecithin may be 20: (8-12), for example 20:10.94.

The amount of the electrically neutral phospholipid may be 30.94%, 37%, 37.44%, 38.25%, 39.08%, 43.5%, 48.43% or 52.17%.

The anionic phospholipid may be a phosphatidic acid and/or a ganglioside. The phosphatidic acid is preferably dimyristylphosphatidic acid (DMPA), distearoyl phosphatidic acid (DSPA), dipalmitoyl phosphatidic acid (DPPA) or dioleoyl phosphatidic acid (DOPA). The ganglioside is preferably monosialotetrahexose ganglioside (GM1).

When the anionic phospholipid is a mixture of GM1 and DOPA, the mass ratio of GM1 to DOPA may be 30.07:(3-7), e.g., 30.07:5.

Herein, the anionic phospholipid may be used in an amount of 24.04%, 25.66%, 26%, 27.22%, 31.5%, 33.06%, 35.07% or 37.72%.

The total amount of hyaluronic acid and protamine used may be 1.15%, 2.35%, 2.58%, 2.63%, 2.68%, 4.17% or 8.6%.

The particle size of the nanocomplex can be 20-95 nm, for example, 27.55±6.99 nm, 36.30±6.41 nm, 37.63±4.20 nm, 37.68±2.20 nm, 39.12±4.84 nm, 40.55±7.66 nm, 55.75±7.69 nm, or 75.29 ± 14.53 nm.

The Zeta potential of the nanocomplex can be -70 to -20 mV, for example, -63.7±2.66, -64.87±3.30, -52.07±2.15, -45.20±2.15, -44.87±0.45 mV, -43.20±2.75 mV, -40.23±6.92, or - 38.27 ± 13.10 mV.

As a preferred embodiment, when the isoelectric point of the protein drug is 4-5.3 and the molecular weight is 200-255 kDa, the nanocomplex comprises 4-17% of protein drug, 0.05-0.25% of hyaluronic acid, 1-3.3% of protamine, 4-16.5% of "apolipoprotein and/or mimetic peptide thereof", 42-53% of electrically neutral phospholipid and 31-38% of anionic phospholipid; wherein the total amount of the hyaluronic acid and the protamine used is 1-3.5%.

The isoelectric point of the protein drug may be 4-4.8, for example, 4.3.

The molecular weight of the protein drug may be 220-250 kDa, for example, 240 kDa.

The protein drug may be phycoerythrin.

The protein drug may be used in an amount of 4.8%, 8%, 8.5%, 11% or 16%.

The hyaluronic acid may be used in an amount of 0.08%, 0.13%, 0.15% or 0.2%.

The protamine may be used in an amount of 1%, 1.5% or 3%.

The "apolipoprotein and/or mimetic peptide thereof" may be used in an amount of 4.12%, 5.8%, 10.35% or 16.37%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, for example, ApoE.

The electrically neutral phospholipid may be phosphatidylcholine. The phosphatidylcholine is preferably DMPC.

The electrically neutral phospholipid may be used in an amount of 42.92%, 43.5%, 46.4%, 47.56% or 52.2%.

The anionic phospholipid may be DOPA.

The anionic phospholipid may be used in an amount of 31.08%, 31.5%, 33.6%, 34.44% or 37.8%.

The total amount of hyaluronic acid and protamine used may be 1.08%, 1.15%, 1.2%, 1.63% or 3.2%.

The particle size of the nanocomplex may be 12-60 nm, such as 20.30±5.89 nm, 23.79±7.91 nm, 27.31±10.84 nm, 28.96±8.74 nm, or 37.55 ± 13.73 nm.

The Zeta potential of the nanocomplex can be -65 to -30 mV, e.g., -58.87±4.90, - 56.33±3.26, -50.10±3.18, -43.93±14.03, or -36.83 ± 2.71 mV.

As a preferred embodiment, when the isoelectric point of the protein drug is 9.3-11 and the molecular weight is 8-16 kDa, the nanocomplex comprises 31.5-34.5% of protein drug, 0.25-0.45% of hyaluronic acid, 0.1-0.35% of protamine, 3-5% of "apolipoprotein and/or mimetic peptide thereof", 35-37.5% of electrically neutral phospholipid and 25-27.5% of anionic phospholipid; wherein the total amount of the hyaluronic acid and the protamine used is 0.5-0.85%.

The isoelectric point of the protein drug may be 9.8-10.8, for example, 10.3.

The molecular weight of the protein drug may be 10-14 kDa, for example, 12.4 kDa.

The protein drug may be cytochrome C.

Here, the protein drug may be used in an amount of 32-34%, for example, 33.07%.

The hyaluronic acid may be used in an amount of 0.3-0.5%, for example, 0.44%.

The protamine may be used in an amount of 0.1-0.3%, for example, 0.2%.

The "apolipoprotein and/or mimetic peptide thereof" may be used in an amount of 3.5-4.5%, for example 4.1%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, for example, ApoE.

The electrically neutral phospholipid may be phosphatidylcholine. The phosphatidylcholine is preferably DMPC.

The electrically neutral phospholipid may be used in an amount of 35-37%, for example, 36.09%.

The anionic phospholipid may be DOPA.

The anionic phospholipid may be used in an amount of 25-27%, for example, 26.1%.

The total amount of the hyaluronic acid and the protamine may be 0.5-0.8%, for example, 0.64%.

The particle size of the nanocomplex may be 25-38 nm, for example, 31.11 ± 3.44 nm.

The Zeta potential of the nanocomplex may be -25 to -15 mV, for example, -21.03 ± 2.47 mV.

As a preferred embodiment, when the isoelectric point of the protein drug is 7-9 and the molecular weight is 140-180 kDa, the nanocomplex comprises 5-43% of protein drug, 0.04-0.9% of hyaluronic acid, 1-14% of protamine, 2.5-20% of "apolipoprotein and/or mimetic peptide thereof", 23.5-46% of electrically neutral phospholipid and 16-32% of anionic phospholipid; wherein the total amount of the hyaluronic acid and the protamine used is 1-13.5%.

The isoelectric point of the protein drug may be 7.5-8.5, for example, 8.

The molecular weight of the protein drug may be 150-170 kDa, for example, 160 kDa.

The protein drug may be an IgG antibody.

The protein drug may be used in an amount of 6%, 20%, 33% or 41%.

The hyaluronic acid may be used in an amount of 0.06%, 0.09%, 0.15%, 0.41% or 0.78%.

The protamine may be used in an amount of 1.3%, 1.7%, 2%, 4.3% or 13%.

The "apolipoprotein and/or mimetic peptide thereof" may be used in an amount of 2.85%, 2.92%, 3.94%, 7.21%, 19.29%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, such as ApoE or ApoA-I mimetic peptides.

The electrically neutral phospholipid may be phosphatidylcholine. The phosphatidylcholine is preferably DMPC.

The electrically neutral phospholipid may be used in an amount of 24.36%, 34.22%, 41.21%, 42.34%, or 45%.

The anionic phospholipid may be DOPA.

The anionic phospholipid may be used in an amount of 17.64%, 24.78%, 29.79%, 30% or 30.66%.

The total amount of hyaluronic acid and protamine used may be 1.71%, 1.79%, 2.15%, 5.08% or 13.06%.

The nanocomplex may have a particle size of 15 to 95 nm, such as 26.52±4.31 nm, 27.38±7.83 nm, 37.98±14.29 nm, or 74.20 ± 14.23 nm.

The Zeta potential of the nanocomplex can be -70 to -20 mV, e.g., -57.33±2.31, - 55.20±10.74, -43.87±9.68, or -31.57 ± 4.67 mV.

As a preferred embodiment, when the isoelectric point of the protein drug is 8.3-10.3 and the molecular weight is 15-40 kDa, the nanocomplex comprises 1-3% of protein drug, 0.2-0.6% of hyaluronic acid, 4.5-6.5% of protamine, 2.5-5% of "apolipoprotein and/or mimetic peptide thereof", 54-57% of electrically neutral phospholipid and 32-35% of anionic phospholipid; wherein the total amount of the hyaluronic acid and the protamine used is 5-7%.

The isoelectric point of the protein drug may be 8.8-9.8, for example, 9.3.

The molecular weight of the protein drug may be 20-33 kDa, for example, 26 kDa.

The protein drug may be a nerve growth factor β-NGF.

The protein drug may be used in an amount of 1.5-2.2%, for example, 1.78%.

The hyaluronic acid may be used in an amount of 0.3-0.5%, for example, 0.43%.

The protamine may be used in an amount of 5-6%, for example, 5.53%.

The "apolipoprotein and/or mimetic peptide thereof" may be used in an amount of 3-4.5%, for example 3.82%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, for example, ApoE.

The electrically neutral phospholipid may be phosphatidylcholine. The phosphatidylcholine is preferably DMPC.

The electrically neutral phospholipid may be used in an amount of 54.5-56%, for example, 55.32%.

The anionic phospholipid may be DOPA.

The anionic phospholipid may be used in an amount of 32.5-34%, for example, 33.12%.

The total amount of the hyaluronic acid and the protamine may be 5.5-6.5%, for example, 5.96%.

As a preferred embodiment, when the isoelectric point of the protein drug is 6-8.5 and the molecular weight is 30-50 kDa, the nanocomplex comprises 15-20% of protein drug, 0.05-0.8% of hyaluronic acid, 0.8-1.6% of protamine, 3-6% of "apolipoprotein and/or mimetic peptide thereof", 44-47% of electrically neutral phospholipid and 29-33% of anionic phospholipid; wherein the total amount of the hyaluronic acid and the protamine used is 0.3-2%.

The isoelectric point of the protein drug may be 6.5-8, for example 7.2.

The molecular weight of the protein drug may be 35-45 kDa, for example, 40 kDa.

The protein drug may be an active enzyme-based protein drug HRP.

The protein drug may be used in an amount of 16.5-19%, for example, 17.73%.

The hyaluronic acid may be used in an amount of 0.1-0.3%, for example, 0.14%.

The protamine may be used in an amount of 0.8-1.5%, for example, 0.83%.

The "apolipoprotein and/or mimetic peptide thereof" may be used in an amount of 4-5%, for example, 4.58%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, for example, ApoE.

The electrically neutral phospholipid may be phosphatidylcholine. The phosphatidylcholine is preferably DMPC.

The electrically neutral phospholipid may be used in an amount of 45-46%, for example, 45.61%.

The anionic phospholipid may be DOPA.

The anionic phospholipid may be used in an amount of 30.5-32%, for example, 31.11%.

The total amount of the hyaluronic acid and the protamine may be 0.5 to 1.5%, for example, 0.97%.

The particle size of the nanocomplex may be 20-57, for example, 37.22 ± 7.28 nm.

The Zeta potential of the nanocomplex may be -55 to -15 mV, for example, -48.87 ± 1.95 mV.

As a preferred embodiment, when the isoelectric point of the protein drug is 4.4-6.4 and the molecular weight is 210-255 kDa, the nanocomplex comprises 10-33% of protein drug, 0.2-3% of hyaluronic acid, 3.5-7% of protamine, 3-6% of "apolipoprotein and/or mimetic peptide thereof", 32-42% of electrically neutral phospholipid and 22-39% of anionic phospholipid; wherein the total amount of the hyaluronic acid and the protamine used is 4-8%.

The isoelectric point of the protein drug may 4.9 and 5.9, for example 5.4.

The molecular weight of the protein drug may be 220-250 kDa.

The protein drug may be catalase (CAT).

The protein drug may be used in an amount of 11-32%, such as 12.06 or 30.9%.

The hyaluronic acid may be used in an amount of 0.3-2.5%, such as 0.5% or 1.53%.

The protamine may be used in an amount of 4-5.5%, such as 4.85% or 5.1%.

The "apolipoprotein and/or mimetic peptide thereof" may be used in an amount of 4-5%, for example, 4.43% or 4.5%.

The "apolipoprotein and/or mimetic peptide thereof" may be one or more of ApoE and mimetic peptides thereof, ApoA-I, ApoA-II, ApoA-IV and mimetic peptides thereof, ApoC-I, ApoC-II, ApoC-III and mimetic peptides thereof, for example, ApoE.

The electrically neutral phospholipid may be phosphatidylcholine. The phosphatidylcholine is preferably DMPC.

The electrically neutral phospholipid may be used in an amount of 33-41%, for example, 34.2% or 39.91%.

The anionic phospholipid may be DOPA.

The anionic phospholipid may be used in an amount of 24-38%, such as 24.8% or 37.22%.

The total amount of the hyaluronic acid and the protamine may be 5-7%, such as 5.6% or 6.38%.

The particle size of the nanocomplex may be 55-70 nm, for example, 63.62 ± 1.97 nm.

The Zeta potential of the nanocomplex may be -25 to -15 mV, for example, -19.43 ± 1.96 mV.

It is a second object of the present invention to provide a preparation method for the nanocomplex, which is prepared by the following method I or method II:
method I
   S1. mixing the protein drug, the hyaluronic acid and the protamine to form a nanogel;
   S2. preparing a liposome using the lipid component by conventional methods;
   S3. co-incubating the nanogel with the liposome to form a nanogel-loading liposome;
   S4. self-assembling the nanogel-loading liposome and the "apolipoprotein and/or the mimetic peptide thereof' to form the nanocomplex;
method II
   S1. mixing the protein drug, the hyaluronic acid and the protamine to form a nanogel;
   S2. preparing a liposome using the lipid component by conventional methods;
   S3. preparing a nanogel-loading liposome by using a microfluidic chip to combine the nanogel with the liposome, and obtaining a liposome loaded with protein drug after removing the solvent by ultrafiltration
   S4. self-assembling the liposome loaded with protein drug and the "apolipoprotein and/or the mimetic peptide thereof' to form the nanocomplex

The operation and method of the mixing in method I or II, S1, may be conventional in the art.

In method I or II, S2, the conventional method may be a method for preparing liposomes conventional in the art, for example, a film hydration method, an injection method, a reconstitution method, a melting method or a freeze-drying method.

As a preferred embodiment, in method one, S2, the liposome is prepared by the following steps. The lipid component is dissolved in an organic solvent, and then the solvent is evaporated off on a rotary evaporator, so that the lipid component forms a thin film on the wall. Then, an appropriate amount of a buffer solution is added, and it is fully hydrated and dispersed by shaking to obtain liposomes. Liposome particle size can be further reduced by ultrasound with a sonicator probe.

As a preferred embodiment, in method II, S2, the liposome is prepared by the following steps. The lipid component is dissolved in ethanol to form liposomes.

In method I, S3, the operation and methods of incubation may be conventional in the art.

In method I or II, S4, the operation and method of self-assembly may be conventional in the art, e.g., the mixture may be incubated at 37°C for 36 hours at 120 rpm on a shaker.

In method II, S3, the method for preparing the nanogel and the liposome used for preparing the liposome containing the nanogel via a microfluidic chip can be a conventional method in the art.

A third object of the present invention is to provide a use of the nanocomplex in the delivery of the protein drug.

As a preferred embodiment, the nanocomplex can be used for intracellular delivery of the protein drug.

As a preferred embodiment, the nanocomplex can be used for in vivo delivery of the protein drug.

As a preferred embodiment, the nanocomplex can be used for intracerebral delivery of the protein drug.

In the present invention, "% "means a mass percentage unless otherwise specified.

The preferred examples of the present invention can be obtained by any combination of the above-mentioned preferred conditions on the basis of common knowledge in the art.

The reagents and starting materials used in the present invention are commercially available.

The positive effect of the present invention is as follows.

The nanocomplex of the present invention includes a specific amount of a protein drug, hyaluronic acid, protamine and a recombinant lipoprotein. A microenvironment suitable for the protein drug is constituted by a proper ratio of hyaluronic acid and protamine. The intracellular, in vivo or even intracerebral delivery of the protein drug is achieved by the recombinant lipoprotein.

The nanocomplex of the present invention provides a general-type carrier for protein drugs having different plysicochemical properties (for example, a molecular weight of 10-255 KDa and PI 4-11), implements highly efficient intracellular and in vivo delivery. The utilized technology is universal in nature. The invention can effectively solve the current problem of insufficient transport in vivo and in vitro of protein drugs (including a protein drug exceeding the molecular weight and PI of an embodiment).

### BRIEF DESCRIPTION OF THE DRAWINGS

Fig. 1 shows a fluorescence distribution diagram of Hela cells incubated with different formulations of bovine serum albumin.
Fig. 2 shows a fluorescence distribution diagram of Hela cells incubated with different formulations of phycoerythrin.
Fig. 3 shows a fluorescence distribution diagram of U87 cells incubated with different formulations of cytochrome C.
Fig. 4 shows a fluorescence distribution diagram of HT22 cells incubated with different formulations of Alexa Fluor488-IgG.
Fig. 5 shows a fluorescence distribution diagram of C6 cells incubated with different formulations of Alexa Fluor488-IgG nanocomplexs.
Fig. 6 shows that a nerve growth factor (NGF)-loaded nanocomplexs promote PC12 cell differentiation. Fig. 6A shows statistics of neurite length of PC12 cells differentiated following the treatment of different formulations; Fig. 6B shows statistics of neurite branch point of PC12 cells differentiated following the treatment of different formulations; Fig. 6C shows the morphology of PC12 cell differentiation promoted by different formulations.
Fig. 7 shows a fluorescence distribution diagram of BV2 cells after uptaking different formulations of catalase.
Fig. 8 shows that CAT-HA-PRTM-rHDL effectively increases intracellular catalase levels (Fig. 8A) and effectively reduces H₂O₂-induced increase in intracellular ROS levels (Fig. 8B).
Fig. 9 shows that CAT-HA-PRTM-rHDL effectively inhibites membrane damage induced by H₂O₂ (Fig. 9A) and effectively increases cell viability (Fig. 9B).
Fig. 10 shows that in the cortical injury model of C57 mice, the fluorescent dye DiR-labelled recombinant lipoprotein nanocomplex (CAT-HA-PRTM-rHDL) loading hyaluronic acid, protamine and CAT and liposome (CAT-HA-PRTM-LIPO) loading hyaluronic acid, protamine and CAT are administered via the tail vein. 4 hours after administration, the organs of mice are taken for living imaging to evaluate the in vivo distribution and brain penetration of the formulations.
Fig. 11 shows that in the cortical injury model of CX3CR1-GFP mice, different formulations labelled with fluorescent dye DiI were administered through the tail vein, and the brain of the mice was frozen sliced 4 h after administration to evaluate the uptake of the CAT-loading nanocomplexes by cerebral microglia in vivo.
Fig. 12 shows that in a cortical injury model of C57 mice and a sham injury control group, administration continued for 1 week after modeling, the effects of different formulations loading CAT on the latency, the times of crossing the platform and the time in the target quadrant of CCI model mice are investigated by the Morris water maze test.
Fig. 13 shows that in the SOD mouse model, the exercise ability of mice was evaluated following continuous administration of drugs, and the effects of different formulations on the exercise ability of SOD transgenic mice were investigated by rod spinning experiment, rod grasping experiment and hind limb gripping experiment.
Fig. 14 shows a fluorescence distribution diagram of Hela cells incubated with the carriers with or without protein payload (Fig. 14A), and shows the intracerebral distribution of carriers with or without protein payload in a cortical injury mice model (Fig. 14B).

### DETAILED DESCRIPTION OF THE INVENTION

The instruments used in the following examples are as follows:
Rotary evaporator (RE-52CS-1, Shanghai Yarong Biochemical Instrument Factory, China)
Ultrasonic apparatus (JY92-II, Ningbo Xinzhi Biotechnology Co. Ltd., China)
Transmission electron microscope (H-7650, Hitachi, japan)
Cryo-electron microscope (FEI Tecnai F20, holland)
Laser granulometer (Zetasizer Nano ZS90 ZEN3590, Malvern, UK)
Microplate reader (Thermo, USA)
Laser confocal microscopy (TCS SP8, leica, Germany).

### Example 1 Preparation, characterization and intracellular protein delivery of nanocomplex loaded with bovine serum albumin

### (1) Preparation

(1.1) Bovine serum albumin, hyaluronic acid and protamine were co-incubated at different mass ratios to form a protein-loaded nanogel (FITC-BSA-HA-PRTM).
(1.2) Preparation of liposomes via the thin film-hydration method: the lipids (neutral phospholipid DMPC and/or lecithin, cationic phospholipid DOTAP, and anionic phospholipid DOPA and/or anionic sphingolipid monosialotetrahexose ganglioside GM1 and/or DMPA, DPPA, DSPA) were weighed and placed in a 500 mL round-bottom flask. The mixture was added with 2 mL diethyl ether, dried to remove the water in the phospholipid, and added with 2 mL chloroform solution. The same was placed on a rotary evaporator, and vacuumized for 1 hours. 4 mL of 0.01 M PBS solution (pH 7.4) was added, and the mixture was shaken intermittently for 10 min in a 40°C water bath until the membrane was hydrated and exfoliated to obtain liposomes. The particle size of the liposomes was further reduced by ultrasound using an ultrasonic probe to obtain liposomes.
(1.3) The liposomes were co-incubated with FITC-BSA-HA-PRTM at different ratios to form a liposome (FITC-BSA-HA-PRTM-LIPO) encapsulating the protein drug (Tables 1-2). The apolipoprotein (ApoE) was added into the above-mentioned liposome solution, gently mixed well, placed on a shaker and incubated at 120 rpm at 37°C for 36 hours to obtain a nanocomplex loaded with bovine serum albumin (FITC-BSA-HA-PRTM-rHDL), wherein the anionic lipid DOPA is replaced by DMPA or DPPA or DSPA to obtain nanocomplexes loaded with bovine serum albumin which are FITC-BSA-HA-PRTM-rHDL-1, FITC-BSA-HA-PRTM-rHDL-2 and FITC-BSA-HA-PRTM-rHDL-3, respectively.

### (2) Characterization

The nanocomplexes loaded with bovine serum albumin were negative stained with phosphotungstic acid, and the morphology was observed by a transmission electron microscopy. After further sample preparation, the structure is observed by the cryoelectroscope. The particle size and zeta potential were measured by dynamic light scattering via a Zetasizer. The drug encapsulating efficiency and loading capacity of the nanocomplexes loaded with bovine serum albumin to the protein drugs were determined by a microplate reader.

### (3) Evaluation of intracellular protein delivery

The intracellular protein delivery of the nanocomplex loaded with bovine serum albumin was observed by a laser confocal microscopy. Human cervical cancer cells, Hela cells, were inoculated at a density of 50,000 cells/well in a confocal dish and cultured for 24 hours. The original culture solution was drawn and discarded, and 500 µL of FITC-labelled protein-loaded formulations were added.

Experimental group: recombinant lipoprotein nanocomplex (FITC-BSA-HA-PRTM-rHDL) with bovine serum albumin (FITC-BSA).

Control groups: (a) Free FITC-labelled BSA protein. (b) The nanogel composed of hyaluronic acid, protamine and FITC-BSA (FITC-BSA-HA-PRTM), namely, the protein-loaded nanogel prepared according to the preparation method of (1.1) of step (1). (c) ABSA-loaded recombinant lipoprotein nanocomplex (FITC-BSA-rHDL) without hyaluronic acid and protamine, prepared by the same method as step (1) except that hyaluronic acid and protamine were not added. (d) A liposome (FITC-BSA-HA-PRTM-LIPO) loaded with hyaluronic acid, protamine and FITC-BSA, and the preparation method thereof was the same as step (1.1) (1.2) of step (1) except that apolipoprotein was not added. (e) FITC-BSA + commercial protein transfection reagent (FITC-BSA-Pulsin). (f) A recombinant lipoprotein nanocomplex containing bovine serum albumin with different anions (FITC-BSA), which was prepared by the same method as that of FITC-BSA-HA-PRTM-rHDL, except that DOPA was replaced by DMPA or DPPA or DSPA, respectively, to obtain the nanocomplex loaded with bovine serum albumin, which were FITC-BSA-HA-PRTM-rHDL-1, FITC-BSA-HA-PRTM-rHDL-2 and FITC-BSA-HA-PRTM-rHDL-3, respectively. The experimental group and the control group (administration concentration: 20 µg/mL, calculated according to the amount of FITC-BSA protein) were incubated at 37°C for 4 hours, respectively. Then it was fixed by 3.7% formaldehyde for 10 min at 37°C, stained for 10 min by Hoechest, and washed for 3 times by PBS. Confocal photography and qualitative observation were performed, and the Image J software was used for semi-quantitative analysis of FITC-BSA uptake.

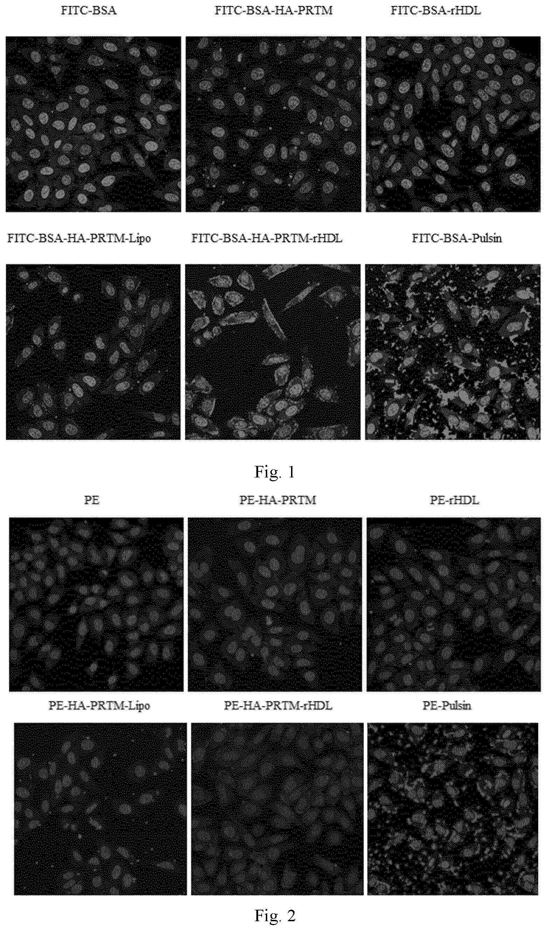

The results show that the particle size of FITC-BSA-HA-PRTM-rHDL is below 100 nm, which is beneficial for the preparation to penetrate the biological membrane barrier. However, the complex of protein, HA and PRTM (FITC-BSA-HA-PRTM) has a larger particle size (198.0 ± 3.10 nm), and further aggregates with time (standing overnight at 4°C), resulting in serious precipitation. When the particle size is detected, the detection instrument indicates that it does not meet the detection requirements and cannot be effectively detected. Compared to FITC-BSA, FITC-BSA-HA-PRTM, FITC-BSA-rHDL, FITC-BSA-HA-PRTM-LIPO, the optimized FITC-BSA-HA-PRTM-rHDL formulation delivered BSAinto cells more efficiently (higher average optical density values of cellular uptake), with the same more evenly distributed in the cytoplasm (as shown in Table 1, Fig. 1). In contrast, while the group of commercially available protein transfection formulation showed stronger cell-associated fluorescence intensities, most of the fluorescence signals were distributed extracellularly (Fig. 1). Meanwhile, protein nanocomplexs formed by different anionic lipids, DMPA or DPPA or DSPA, may efficiently deliver proteins to the cytoplasm of cells. Unexpectedly, in the FITC-BSA-HA-PRTM-rHDL formulation, cellular uptake of protein was lower in the cationic lipid DOTAP-containing formulations (Formula 1) than that in the anionic lipid-containing formulations (Table 2).

**Table 1 Characterization of BSA-loaded nanocomplexs in different formulations and quantification data of Hela cell for uptaking proteins**

| Formulation composition | DMP C (%) | DOP A (%) | DMP A (%) | DP PA (%) | DSPA (%) | FITC-BSA (%) | HA (%) | PRTM (%) | ApoE (%) | Size (nm) | Zeta potent ial (mV) | Cell uptake mean optical density value |
|---|---|---|---|---|---|---|---|---|---|---|---|---|
| FITC-BSA | 0 | 0 | / | / | I | 100% | 0 | 0 | 0 | Not detected | Not detect ed | 1.80 ± 0.19 (***) |
| FITC-BSA-HA-PRTM | 0 | 0 | / | / | I | 92.74 | 1.08 | 6.18 | 0 | 198.0 ± 3.10 | 5.94 ± 0.26 | 2.26 ± 0.09 (***) |
| FITC-BSA-rHDL | 37.89 | 26.63 | / | / | I | 30.72 | 0 | 0 | 4.76 | Not detected | Not detect ed | 1.00 ± 0.00 (***) |
| FITC-BSA-HA-PRTM-LIPO | 38.8 | 27.27 | / | / | I | 31.46 | 0.37 | 2.1 | 0 | 24.63 ± 6.86 | 36.93 ± 3.59 | 2.64 ± 0.15 (***) |
| FITC-BSA-HA-PRTM-rHDL | 37 | 26 | / | / | I | 30 | 0.35 | 2 | 4.65 | 27.55 ± 6.99 | 45.20 ± 2.15 | 7.24 ± 0.19 (###) |
| FITC-BSA-HA-PRTM-rHDL-1 | 39.08 | 0 | 24.04 | 0 | 0 | 29.31 | 0.24 | 2.44 | 4.89 | 55.75 ± 7.69 | 64.87 ± 3.30 | 6.35 ± 0.06 (###) |
| FITC-BSA-HA-PRTM-rHDL-2 | 38.25 | 0 | 0 | 25. 66 | 0 | 28.68 | 0.24 | 2.39 | 4.78 | 37.68 ± 2.20 | - 44.87 ± 0.45 | 6.14 ± 0.05 (###) |
| FITC-BSA-HA-PRTM-rHDL-3 | 37.44 | 0 | 0 | 0 | 27.22 | 28.08 | 0.24 | 2.34 | 4.68 | 40.55 ± 7.66 | - 43.20 ± 2.75 | 6.20 ± 0.08 (###) |

| | | | | | | | | | | | | |
|---|---|---|---|---|---|---|---|---|---|---|---|---|
| notes: (a) The formulation composition of FITC-BSA-HA-PRTM-rHDL in Table 1 is the formulation composition of Formulation 6 in Table 2. (b) "Not detected" means relevant detection is not made. (c) The Zeta potential represents the potential of the nanoparticles, which is determined by the material for constituting the nanocarrier. (d) * * * has a significant difference compared with FITC-BSA-HA-PRTM-rHDL group; # # # is significantly different from the FITC-BSA group. | | | | | | | | | | | | |

Fig. 1 shows the uptake of different formulations of bovine serum albumin in Hela cells. Green: FITC-labelled bovine serum albumin (FITC-BSA); FITC-BSA-HA-PRTM-rHDL: a recombinant lipoprotein nanocomplex loaded with hyaluronic acid, protamine, and FITC-BSA; FITC-BSA-HA-PRTM: the complex of hyaluronic acid, protamine, and FITC-BSA; FITC-BSA-rHDL: the recombinant lipoprotein nanocomplex loaded with BSA and without hyaluronic acid and protamine; FITC-BSA-HA-PRTM- LIPO: a liposome loaded with hyaluronic acid, protamine, and FITC-BSA; FITC-BSA-Pulsin: the commercial protein transfection reagent with BSA.

**Table 2 Characterization of BSA-loaded nanocomplexs with different FITC-BSA-HA-PRTM-rHDL formulations and quantification data of C6 cell uptaking of proteins**

| Formul ation compos ition | DMP C (%) | Lecith in (%) | DOPA, (%) | GM1 (%) | DOT AP (%) | EPC (%) | FITC-BSA (%) | HA (%) | PRT M (%) | ApoE (%) | Size (nm) | Zeta (mV) | Cell uptake mean optical density value | Formul ation stabilit Y |
|---|---|---|---|---|---|---|---|---|---|---|---|---|---|---|
| Formul ation 1 | 47.06 | 0 | 0 | 0 | 35.29 | 0 | 9.41 | 4.7 | 1.19 | 2.35 | 84.69 ± 11.42 | 22.10 ± 0.17 | 2.00 ± 0.00 | Stable |
| Formul ation 2 | 40 | 0 | 0 | 0 | 0 | 23 | 25 | 0.5 | 4 | 7.5 | 68.15 ± 31.06 | 25.13 ± 1.70 | 2.57 ± 0.05 (***) | Stable |
| Formul ation 3 | 43.5 | 0 | 31.5 | 0 | 0 | 0 | 16 | 0.47 | 3.7 | 4.83 | 75.29 ± 14.53 | -38.27 ± 13.10 | 3.38 ± 0.04 (***) | Stable |
| Formul ation 4 | 48.43 | 0 | 30.07 | 5 | 0 | 0 | 9 | 0.15 | 1 | 6.35 | 37.63 ± 4.20 | -63.7 ± 2.66 | 3.58 ± 0.04 (***) | Stable |
| Formul ation 5 | 20 | 10.94 | 33.06 | 0 | 0 | 0 | 22 | 2.1 | 6.5 | 5.4 | 39.12 ± 4.84 | -40.23 ± 6.92 | 3.50 ± 0.00 (***) | Stable |
| Formul ation 6 | 37 | 0 | 26 | 0 | 0 | 0 | 30 | 0.35 | 2 | 4.65 | 27.55 ± 6.99 | -45.20 ± 2.15 | 4.00 ± 0.00 (***) | Stable |
| Formul ation 7 | 43.96 | 0 | 21.98 | 0 | 0 | 0 | 8.79 | 2.2 | 17.5 8 | 5.49 | 281 | 23.4 | Not detected | Easy settlem ent |

| | | | | | | | | | | | | | | |
|---|---|---|---|---|---|---|---|---|---|---|---|---|---|---|
| Notes: (a) Formulations 1, 2, and 7 in Table 2 are control formulations and Formulations 3-6 are example formulations of the present invention. (b) The stability evaluation method for formulations is storing overnight at 4°C for 12h. (c) EPC component is a cationic lipid. In Formulation 7, "easy settlement" in the formulation stability refers to the settlement visible to the naked eye when stored at 4°C for 12 hours, and the particle size increases to 1,000 nm or more. (d) Formulations 3-6 have good stability, the particle size and zeta potential are stable after storing at 4°C for 1 month, and there is no change compared with those before storing at 4°C. (e) Statistical analysis is performed with Formulation 1 as control, and * * * P <0.001 is significantly different from Formulation 1. (f) In Table 2, lecithin refers to egg yolk lecithin, which is equivalently replaced with hydrogenated soy lecithin, with same effects. | | | | | | | | | | | | | | |

As can be seen from Table 2, among the Formulations 3-6, the average optical density value of cellular uptake of Formulation 6 is the maximum, indicating that the cellular uptake efficiency is the highest and the effect is the best.

### Example 2 Preparation, characterization and intracellular protein delivery of phycoerythrin-loaded nanocomplexs

### (1) Preparation

(1.1) The phycoerythrin (PE) was co-incubated with hyaluronic acid and protamine at different mass ratios to form a protein-loaded complex (PE-HA-PRTM);
(1.2) Liposomes were prepared by dissolving appropriate amount of phospholipid (DMPC and DOPA) in an ethanol phase;
(1.3) An appropriate amount of PE-HA-PRTM and the above-mentioned liposomes were taken for preparing a protein-loaded liposome (PE-HA-PRTM-LIPO) via a microfluidic chip, with the ethanol solution removed by ultrafiltration. The liposomes with the ethanol removed were incubated with lipoprotein and/or its mimetic peptides had self-assembly to obtain PE-loaded nanocomplexs (PE-HA-PRTM-rHDL) composed of hyaluronic acid, protamine, and recombinant lipoprotein.

### (2) Characterization

The PE-loaded nanocomplexes were negative stained with phosphotungstic acid, and the morphology was observed by transmission electron microscopy. After further sample preparation, the structure was observed by the cryoelectroscope. The particle size and zeta potential were measured by dynamic light scattering via a Zetasizer. The drug encapsulating efficiency and loading capacity of the PE-loaded nanocomplexes were determined by a microplate reader.

### (3) Evaluation of intracellular protein delivery efficiency

The intracellular protein delivery of the PE-loaded nanocomplex was observed by a laser confocal microscopy. Human cervical cancer cells, Hela cells, or glioma cells C6 were inoculated at a density of 50,000/well in a confocal dish and cultured for 24 hours. The original culture solution was drawn and discarded, and 500 µL of PE-loaded recombinant lipoprotein nanocomplex (PE-HA-PRTM-rHDL) was added. Free PE in the control group; a hyaluronic acid, protamine and PE complex (PE-HA-PRTM); a PE-carried recombinant lipoprotein nanocomplex (PE-rHDL) without hyaluronic acid and protamine; and a liposome (PE-HA-PRTM-LIPO) carried with a hyaluronic acid, protamine and PE and PE complex + commercial protein transfection reagent (PE-Pulsin) (administration concentration: 10 µg/mL, calculated according to PE mass) were incubated at 37°C for 4 hours. Then it was fixed by 3.7% formaldehyde for 10 min at 37°C, stained for 10 min by Hoechest, and washed for 3 times by PBS. Confocal photography and qualitative observation were performed, and the Image J software was used for semi-quantitative analysis of FITC-PE uptake.

The results show that the particle size of PE-HA-PRTM-rHDL in a proper proportion is below 100 nm (Tables 3-4), while the protein-carried nanogel (PE-HA-PRTM) composed of hyaluronic acid, protamine and PE has a large particle size (788.1 ± 60.98 nm) and further aggregates with time (storing overnight at 4°C), resulting in serious precipitation. When the particle size is detected, the detection instrument indicates that it does not meet the detection requirements and cannot be effectively detected. Compared to PE, PE-HA-PRTM, PE-rHDL, and PE-HA-PRTM-LIPO, the optimized PE-HA-PRTM-rHDL formulation delivered PE into cells more efficiently, and more evenly distributed in the cytoplasm (Table 3, Fig. 2). Similarly, while the group of commercially available protein transfection formulations showed stronger cell-associated fluorescence intensities, most of the fluorescence signals were distributed extracellularly (Fig. 2). Similarly, in the PE-HA-PRTM-rHDL formulation, cellular uptake of the cationic lipid-containing formulation protein was lower than that of the anionic lipid-containing formulation.

**Table 3 Characterization of phycoerythrin nanocomplexs in different formulations and quantification data of Hela cell for uptaking proteins**

| Formulation composition | DMPC (%) | DOPA (%) | PE (%) | HA (%) | PRTM (%) | ApoE (%) | Size (nm) | Zeta potential (mV) | Cell uptake mean optical density value |
|---|---|---|---|---|---|---|---|---|---|
| PE | 0 | 0 | 100 | 0 | 0 | 0 | Not detected | Not detected | 1.50 ± 0.00 (***) |
| PE-HA-PRTM | 0 | 0 | 90.15 | 1.64 | 8.21 | 0 | 788.1 ± 60.98 | 9.15 ± 0.24 | 1.78 ± 0.08 (***) |
| PE-rHDL | 48.15 | 34.85 | 11.13 | 0 | 0 | 5.87 | 40.81 ± 4.0 | -40.73 ± 3.91 | 1.48 ± 0.04 (***) |
| PE-HA-PRTM-LIPO | 50.49 | 36.56 | 11.68 | 0.21 | 1.06 | 0 | 34.59 ± 8.90 | -35.93 ± 6.49 | 1.96 ± 0.09 (***) |
| PE-HA-PRTM-rHDL | 47.56 | 34.44 | 11 | 0.2 | 1 | 5.8 | 29.66 ± 3.91 | -42.83 ± 4.70 | 4.07 ± 0.10 |

| | | | | | | | | | |
|---|---|---|---|---|---|---|---|---|---|
| Notes: "Not detected" means relevant detection is not made. Statistical analysis is performed with PE-HA-PRTM-rHDL as control, and * * * P < 0.001 is significantly different from PE-HA-PRTM-rHDL. | | | | | | | | | |

Fig. 2 shows the conditions of Hela cells for uptaking different formulations of phycoerythrin. Phycoerythrin (PE); a recombinant lipoprotein nanocomplex (PE-HA-PRTM-rHDL) carried with hyaluronic acid, protamine and PE; a hyaluronic acid, protamine and PE complex (PE-HA-PRTM); a PE-carried recombinant lipoprotein nanocomplex (PE-rHDL) without hyaluronic acid and protamine; a liposome (PE-HA-PRTM-LIPO) carried with hyaluronic acid, protamine and PE and PE + commercial protein transfection reagent (PE-Pulsin).

**Table 4 Characterization of phycoerythrin-loaded nanocomplexs at different PE-HA-PRTM-rHDL components ratio and quantification data of C6 cell for uptaking proteins**

| Formulation composition | DMPC | DOPA | DOTAP | PE | HA | PRTM | ApoE | Size (nm) | Zeta (mV) | Cell uptake mean optical density value | Formulation stability |
|---|---|---|---|---|---|---|---|---|---|---|---|
| | (%) | (%) | (%) | (%) | (%) | (%) | (%) | | | | |
| Formulation 1 | 47.56 | 0 | 34.44 | 11 | 0.2 | 1 | 5.8 | 25.42 ± 4.32 | 21.72 ± 3.46 | 1.72 ± 0.08 | Stable |
| Formulation 2 | 11.2 | 10.17 | - | 60.95 | 3.31 | 11.4 | 2.97 | 423.57 57.34 | Not detected | 1.64±0.05 (ns) | Easy settlement |
| Formulation 3 | 43.5 | 31.5 | 0 | 16 | 0.2 | 3 | 5.8 | 27.31 10.84 | -36.83 ± 2.71 | 2.44±0.05 (***) | Stable |
| Formulation 4 | 52.2 | 37.8 | 0 | 4.8 | 0.08 | 1 | 4.12 | 28.96 ± 8.74 | -58.87 ± 4.90 | 2.60±0.00 (***) | Stable |
| Formulation 5 | 46.4 | 33.6 | 0 | 8.5 | 0.15 | 1 | 10.35 | 37.55 ± 13.73 | -50.10 ± 3.18 | 2.56±0.05 (***) | Stable |
| Formulation 6 | 42.92 | 31.08 | 0 | 8 | 0.13 | 1.5 | 16.37 | 23.79 ± 7.91 | -56.33 ± 3.26 | 2.00±0.00 (***) | Stable |
| Formulation 7 | 47.56 | 34.44 | 0 | 11 | 0.2 | 1 | 5.8 | 20.30 ± 5.89 | -43.93 ± 14.03 | 4.54±0.05 (***) | Stable |

| | | | | | | | | | | | |
|---|---|---|---|---|---|---|---|---|---|---|---|
| Notes: (a) In Table 4, Formulations 1 and 2 are control formulations and Formulations 3-7 are example formulations of the present invention. (b) The stability evaluation method for preparation is letting stand at 4°C for 12h. (c) In Formulation 2, "easy settlement" refers to the settlement visible to the naked eye when stored at 4°C for 12 hours, and the particle size increases to 1,000 nm or more. (d) Formulations 3-7 have good stability, the particle size and potential are stable after storing at 4°C for 1 month, and there is no change compared with those before storing. (e) Statistical analysis is performed with Formulation 1 as control, and * * * P <0.001 is significantly different from Formulation 1. | | | | | | | | | | | |

As can be seen from Table 4, among the Formulations 3-7, the average optical density value of cellular uptake of Formulation 7 is the maximum, indicating that the cellular uptake efficiency is the highest and the effect is the best.

### Example 3 Preparation, characterization and intracellular protein delivery of cytochrome C-loaded nanocomplexs

### (1) Preparation

(1.1) The fluorescent labelled cytochrome C was co-incubated with hyaluronic acid and protamine at different mass ratios to form a protein-loaded complex (FITC-CC-HA-PRTM);
(1.2) Preparation of liposomes by membrane hydration: the lipids (a combination of neutral phospholipid DMPC and anionic phospholipid DOPA) were weighed and placed in a 500 mL round-bottom flask. The mixture was added with 2 mL diethyl ether, dried to remove the water in the phospholipid, and added with chloroform solution. The same was placed on a rotary evaporator, and vacuumized for 1 hours. 4 mL of 0.01 M PBS solution (pH 7.4) was added, and the mixture was shaken intermittently for 10 min in a 40°C water bath until the membrane was hydrated and exfoliated to obtain liposomes. The particle size of the liposomes was further reduced by ultrasound using a probe to obtain liposomes.
(1.3) the liposomes prepared above were incubated with different ratios to form a liposome (FITC-CC-HA-PRTM-LIPO) encapsulating the protein drug. ApoE was added to the nanocomplex solution, gently mixed, and incubated on a shaker at 120 rpm at 37°C for 36 hours to obtain a cytochrome C-carried nanocomplex (FITC-CC-HA-PRTM-rHDL).

### (2) Characterization

The morphology of cytochrome C-loaded nanocomplexs was observed by phosphotungstic acid negative staining and transmission electron microscopy. After further sample preparation, the structure was observed by the cryoelectroscope. The particle size and zeta potential were measured by a laser granulometry. The encapsulating efficiency and drug carrying capacity of the cytochrome C-carried nanocomplexes to the protein drugs were determined by a microplate reader.

### (3) Evaluation of intracellular protein delivery

The intracellular protein delivery of the cytochrome C-carried nanocomplex was observed by a laser confocal microscopy. The glioma cell line U87 was inoculated at a density of 50,000/well in a confocal dish and cultured for 24 hours. The original culture solution was drawn and discarded, 500 µL of recombinant lipoprotein nanocomplex (FITC-CC-HA-PRTM-rHDL) loaded with green fluorescent protein labelled cytochrome C (FITC-CC) was added, and the free FITC-CC proteins in the control group, respectively; a Hyaluronic acid, protamine, and FITC-CC complex (FITC-CC-HA-PRTM); a CC-carried recombinant lipoprotein nanocomplex (FITC-CC-rHDL) without hyaluronic acid and protamine; liposomes (FITC-CC-HA-PRTM- LIPO) carried with hyaluronic acid, protamine, and FITC-CC and FITC-CC + commercial protein transfection reagent (FITC-CC-Pulsin) (administration concentration: 20 µg/mL, calculated according to the amount of FITC-CC protein) were incubated at 37°C for 6 hours. Then it was fixed by 3.7% formaldehyde for 10 min at 37°C, stained for 10 min by Hoechest, and washed for 3 times by PBS. Confocal photography and qualitative observation were performed, and the Image J software was used for semi-quantitative analysis of FITC-CC uptake.

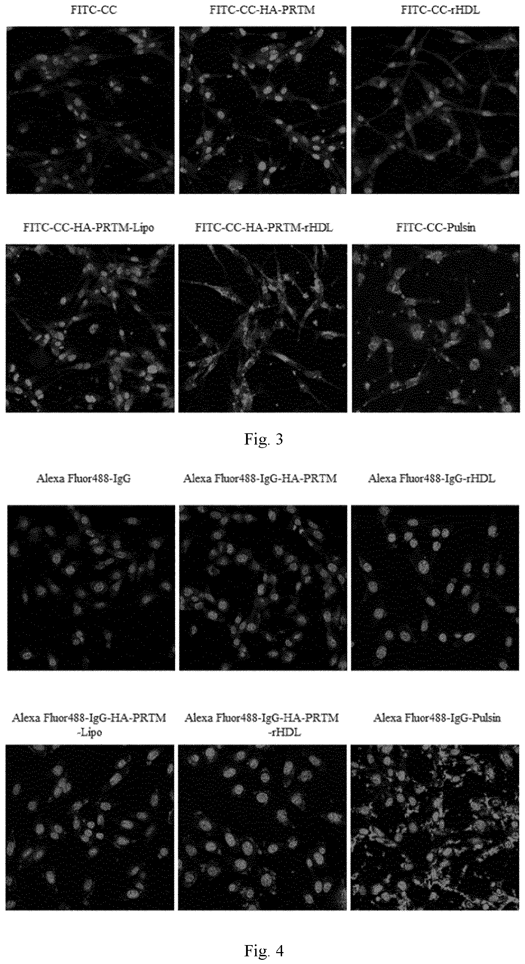

The results show that the particle size of FITC-CC-HA-PRTM-rHDL at a appropriate proportion is below 100 nm. Compared to FITC-CC, FITC-CC-HA-PRTM, FITC-CC-rHDL, FITC-CC-HA-PRTM-LIPO and the commercial protein transfection formulation groups, the optimized FITC-CC-HA-PRTM-rHDL formulation delivered CC more efficiently into cells, with same being more evenly distributed in the cytoplasm (Table 5, Fig. 3).

**Table 5 Characterization of cytochrome C in different formulations and quantification data of U87 cellular for uptaking proteins**

| Formulation composition | DMPC (%) | DOP A (%) | FITC -CC (%) | HA (%) | PRTM (%) | ApoE (%) | Size (nm) | Zeta potential (mV) | Cell uptake mean optical density value |
|---|---|---|---|---|---|---|---|---|---|
| FITC-CC | 0 | 0 | 100 | 0 | 0 | 0 | Not detected | Not detected | 1.40 ± 0.00 (***) |
| FITC-CC-HA-PRTM | 0 | 0 | 98.1 | 1.31 | 0.59 | 0 | 52.72 ± 90.13 | -1.60 ± 0.64 | 2.96 ± 0.11 (***) |
| FITC-CC-rHDL | 36.32 | 26.27 | 33.2 8 | 0 | 0 | 4.13 | 24.47 ± 11.89 | -29.27 ± 2.73 | 1.32 ± 0.04 (***) |
| FITC-CC-HA-PRTM-LIPO | 37.63 | 27.22 | 34.4 8 | 0.46 | 0.21 | 0 | 33.66 ± 5.60 | -12.30 ± 2.89 | 4.20 ± 0.16 (***) |
| FITC-CC-HA-PRTM-rHDL | 36.09 | 26.1 | 33.0 7 | 0.44 | 0.2 | 4.1 | 31.11 ± 3.44 | -21.03 ± 2.47 | 8.96 ± 0.57 |

| | | | | | | | | | |
|---|---|---|---|---|---|---|---|---|---|
| Notes: (a) "Not detected" means relevant detection is not made. (b) In the formulation composition, FITC-CC-HA-PRTM-rHDL has good stability. The particle size and potential are stable after standing at 4°C for 1 month, and there is no change compared with those before standing. (c) Statistical analysis is performed with FITC-CC-HA-PRTM-rHDL as control. *** P < 0.001 is significantly different from the FITC-CC-HA-PRTM-rHDL group. | | | | | | | | | |

Fig. 3 shows a fluorescence distribution diagram of U87 cells for uptaking different formulations of cytochrome C. Green fluorescent protein labelled cytochrome C (FITC-CC); a recombinant lipoprotein nanocomplex (FITC-CC-HA-PRTM-rHDL) carried with a hyaluronic acid, protamine, and FITC-CC complex; a hyaluronic acid, protamine, and FITC-CC complex (FITC-CC-HA-PRTM); a FITC-CC-carried recombinant lipoprotein nanocomplex (FITC-CC-rHDL) without hyaluronic acid and protamine; a liposome (FITC-CC-HA-PRTM- LIPO) carried with hyaluronic acid, protamine, and FITC-CC + commercial protein transfection reagent (FITC-CC-Pulsin).

### Example 4 Preparation, characterization and intracellular protein delivery of IgG antibody-encapsulated nanocomplexs

### (1) Preparation

(1.1) The fluorescent labelled antibody Alexa Fluor488-IgG was co-incubated with protamine and protamine at different mass ratios to form a protein-carried complex (Alexa Fluor488-IgG-HA-PRTM).
(1.2) Preparation of liposomes by membrane hydration: the lipids (a combination of amphoteric phospholipid DMPC, cationic phospholipid DOTAP and anionic phospholipid DOPA) were weighed and placed in a 500 mL round-bottom flask. The mixture was added with 2 mL diethyl ether, dried to remove the water in the phospholipid, and added with chloroform solution. The same was placed on a rotary evaporator, and vacuumized for 1 hour. 4 mL of 0.01 M PBS solution (pH 7.4) was added, and the mixture was shaken intermittently for 10 min in a 40°C water bath until the membrane was hydrated and exfoliated to obtain liposomes. The particle size of the liposomes was further reduced by ultrasound using a probe to obtain liposomes.
(1.3) The liposomes prepared above were incubated with Alexa Fluor488-IgG-HA-PRTM at different ratios to form a liposome (Alexa Fluor488-IgG-HA-PRTM-LIPO) encapsulating the protein drug. ApoE or ApoA I mimetic peptide (Ac-FAEKFKEAVKDYFAKFWD) was added to the protein-carried liposome solution, gently mixed, and incubated on a shaker at 120 rpm at 37°C for 36 hours to obtain an Alexa Fluor488-IgG antibody-carried nanocomplex (Alexa Fluor488-IgG-HA-PRTM-rHDL).

### (2) Characterization

The Alexa Fluor488-IgG antibody-carried nanocomplexes were negative stained with phosphotungstic acid, and the morphology was observed by a transmission electron microscopy. After further sample preparation, the structure was observed by the cryoelectroscope. The particle size and surface potential were measured by a laser granulometry. The encapsulating efficiency and drug carrying capacity of the Alexa Fluor488-IgG-carried nanocomplexes to the protein drugs were determined by a microplate reader.

### (3) Evaluation of intracellular protein delivery

The intracellular protein delivery of the Alexa Fluor488-IgG-carried nanocomplex was observed by a laser confocal microscopy. Human cervical cancer cells, Hela cells, were inoculated at a density of 50,000 cells/well in a confocal dish and cultured for 24 hours. The original culture solution was drawn and discarded, 500 µL of recombinant lipoprotein nanocomplex (Alexa Fluor488-IgG-HA-PRTM-rHDL) carried with Alexa Fluor488-IgG antibody (Alexa Fluor488-IgG) was added, and the free Alexa Fluor488-IgG protein was added to the control group respectively; a hyaluronic acid, protamine, Alexa Fluor488-IgG complex (Alexa Fluor488-IgG-HA-PRTM); an Alexa Fluor488-IgG-carried recombinant lipoprotein nanocomplex (Alexa Fluor488-IgG-rHDL) without hyaluronic acid and protamine; an Alexa Fluor488-IgG-carried recombinant lipoprotein nanocomplex (Alexa Fluor488-IgG-rHDL) without hyaluronic acid and protamine; a liposome (Alexa Fluor488-IgG-HA-PRTM-LIPO) carried with hyaluronic acid, protamine and Alexa Fluor488-IgG and Alexa Fluor488-IgG + commercial protein transfection reagent (Alexa Fluor488-IgG-Pulsin) (administered at a concentration of 10 µg/mL, calculated as the amount of Alexa Fluor488-IgG protein) were incubated at 37°C for 6 hours. Then it was fixed by 3.7% formaldehyde for 10 min at 37°C, stained for 10 min by Hoechest, and washed for 3 times by PBS. Confocal photography and qualitative observation were performed, and the Image J software was used for semi-quantitative analysis of Alexa Fluor488-IgG uptake.

The results show that the particle size of Alexa Fluor488-IgG at an appropriate proportion is below 100 nm. The complex (Alexa Fluor488-IgG-HA-PRTM) composed of hyaluronic acid, protamine and antibody has a particle size of 63.48 ± 5.20 nm, which aggregates with time (standing overnight at 4°C) and precipitates seriously. The instrument for particle size detection fails to meet the detection requirements and cannot be detected. Compared to Alexa Fluor488-IgG, Alexa Fluor488-IgG-HA-PRTM, Alexa Fluor488-IgG-rHDL and Alexa Fluor488-IgG-HA-PRTM-LIPO, the optimized Alexa Fluor488-IgG-HA-PRTM-rHDL formulation delivered Alexa Fluor488-IgG more efficiently into cells, with the same being more evenly distributed in the cytoplasm (Table 6, Fig. 4). In contrast, while the group of commercially available protein transfection formulations showed stronger cell-associated fluorescence intensities, most of the fluorescence signals were distributed extracellularly.

**Table 6 Characterization of Alexa Fluor488-IgG-carried nanocomplexs in different formulations and quantification data of Hela cellular for uptaking proteins**

| Formulation composition | DMPC (%) | DOPA (%) | Alexa Fluor488 -IgG (%) | HA (%) | PRTM (%) | ApoE (%) | ApoA I mimetic peptide (%) | Size (nm) | Zeta potential (mV) | Cell uptake mean optical density value |
|---|---|---|---|---|---|---|---|---|---|---|
| Alexa Fluor488-IgG | 0 | 0 | 100 | 0 | 0 | 0 | 0 | Not detect ed | Not detected | 1.76 ± 0.05 (***) |
| Alexa Fluor488-IgG-HA-PRTM | 0 | 0 | 91.79 | 0.41 | 7.8 | 0 | 0 | 53.48 ± 5.20 | 4.83 ± 1.25 | 1.80 ± 0.00 (***) |
| Alexa Fluor488-IgG-rHDL | 41.96 | 30.33 | 20.37 | 0 | 0 | 7.34 | 0 | 34.07 ± 4.85 | -58.03 ± 3.51 | 1.67 ± 0.05 (***) |
| Alexa Fluor488-IgG-HA-PRTM-LIPO | 44.41 | 32.1 | 21.56 | 0.1 | 1.83 | 0 | 0 | 30.84 ± 4.98 | -53.90 ± 1.71 | 2.74 ± 0.09 (***) |
| Alexa Fluor488-IgG-HA-PRTM-rHDL-1 | 41.21 | 29.79 | 20 | 0.09 | 1.7 | 7.21 | 0 | 27.38 ± 7.83 | -55.20 ± 10.74 | 4.38 ± 0.08 |
| Alexa Fluor488-IgG-HA-PRTM-rHDL-2 | 45 | 30 | 20 | 0.15 | 2 | 0 | 2.85 | 26.52 ± 4.31 | -57.33 ± 2.31 | 4.10 ± 0.05 (ns) |

| | | | | | | | | | | |
|---|---|---|---|---|---|---|---|---|---|---|
| Notes: (a) "Not detected" means relevant detection is not made. (b) In the formulation composition, the two formulations of Alexa Fluor488-IgG-HA-PRTM-rHDL have good stability. The particle size and potential are at a formulation stable after standing at 4°C for 1 month, and there is no change compared with those before standing. (c) In the statistical analysis, with Alexa Fluor488-IgG-HA-PRTM-rHDL-1 as control, there is significant difference between * * * P < 0.001 and Alexa Fluor488-IgG-HA-PRTM-rHDL-1 group. | | | | | | | | | | |

Fig. 4 shows a fluorescence distribution diagram of HT22 cells for uptaking different formulations of Alexa Fluor488-IgG. Alexa Fluor488-IgG; a recombinant lipoprotein nanocomplex (Alexa Fluor488-IgG-HA-PRTM-rHDL) carried with hyaluronic acid, protamine and Alexa Fluor488-IgG; a hyaluronic acid, protamine and Alexa Fluor488-IgG complex (Alexa Fluor488-IgG-HA-PRTM); an Alexa Fluor488-IgG-carried recombinant lipoprotein nanocomplex (Alexa Fluor488-IgG-rHDL) without hyaluronic acid and protamine; a liposome (Alexa Fluor488-IgG-HA-PRTM-LIPO) carried with hyaluronic acid, protamine and Alexa Fluor488-IgG and Alexa Fluor488-IgG + commercial protein transfection reagent (Alexa Fluor488-IgG-Pulsin).

**Table 7 Characterization of nanocomplexs in different Alexa Fluor488-IgG-HA-PRTM-rHDL formulations and quantification data of C6 cellular uptaking of proteins**

| Formulation composition | DMPC (%) | DOPA (%) | Alexa Fluor488-IgG (%) | HA (%) | PRTM (%) | ApoE (%) | Size (nm) | Zeta potential (mV) | Cell uptake mean optical density value |
|---|---|---|---|---|---|---|---|---|---|
| Formulation 1 | 41.21 | 29.79 | 20 | 0.09 | 1.7 | 7.21 | 37.98 ± 14.29 | -43.87 ± 9.68 | 5.03 ± 0.08 (****) |
| Formulation 2 | 42.34 | 30.66 | 6 | 0.41 | 1.3 | 19.29 | 27.38 ± 7.83 | -55.20 ± 10.74 | 4.63 ± 0.14 (****) |
| Formulation 3 | 34.22 | 24.78 | 33 | 0.78 | 4.3 | 2.92 | 74.20 ± 14.23 | -31.57 ± 4.67 | 3.30 ± 0.09 (****) |
| Formulation 4 | 24.36 | 17.64 | 41 | 0.06 | 13 | 3.94 | Not detected | Not detected | 3.34 ± 0.09 (****) |
| Formulation 5 | 28.77 | 16.83 | 25.3 | 6.53 | 10.4 | 12.17 | 28.82 ± 0.96 | -73.90 ± 2.99 | 2.16 ± 0.05 |

In the table, Formulation 5 is a control group, and Formulations 1-4 are examples of the present invention. *** P < 0.001, * * * * P < 0.0001 is significantly different from Formulation 5.

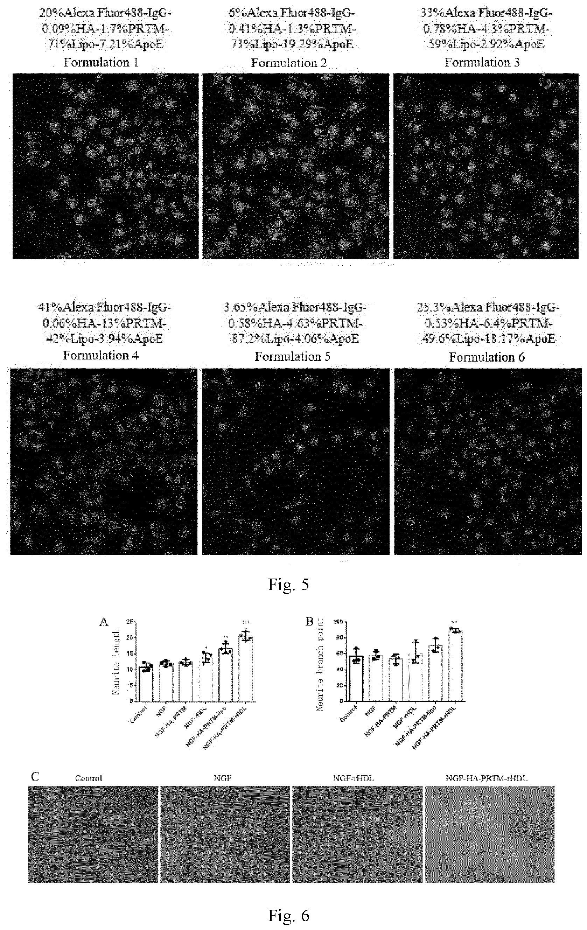

Fig. 5 shows a fluorescence distribution diagram of C6 cells for uptaking different formulations of Alexa Fluor488-IgG nanocomplexs.

### Example 5 Evaluation of the ability of β-NGF-carried nanocomplexs to promote differentiation of PC12 cells

β-NGF was selected as a model for the study of neurotrophic protein drugs, and the same preparation method as in Example 1 was used to prepare the experimental group of β-NGF-carried nanocomplexes and the control group, respectively, and the formulations of the experimental and control groups were shown in Table 8.

Mouse primary neurons or PC12 cells were selected and inoculated in 96-well plates, β-NGF was administered at a concentration of 100 ng/mL, and different carrier protein preparations were co-incubated with the cells for 8 h. The process of β-NGF-induced differentiation of PC12 cells into neurons was detected by the incucyte real-time dynamic cell imaging technique, and the dynamic changes of branching points and synaptic length of neurons induced by the PC12 cells were analyzed semi-quantitatively.

**Table 8 Characterization of NGF-carried nanocomplexes in different formulations and detection of their promoted neurogenic differentiation activity**

| Formulation composition | DMPC (%) | DOPA (%) | NGF (%) | HA (%) | PRTM (%) | ApoE (%) | NGF-promoting neurogenic differentiation activity detection |
|---|---|---|---|---|---|---|---|
| NGF | 0 | 0 | 100 | 0 | 0 | 0 | 12.06 ± 0.76 (***) |
| NGF-HA-PRTM | 0 | 0 | 23 | 5.56 | 71.44 | 0 | 12.55 ± 0.84 (***) |
| NGF-rHDL | 58.64 | 35.1 | 1.88 | 0 | 0 | 4.38 | 13.82 ± 1.49 (**) |
| NGF-HA-PRTM-LIPO | 57.53 | 34.44 | 1.85 | 0.45 | 5.73 | 0 | 16.72 ± 1.54 (*) |
| NGF-HA-PRTM-rHDL | 55.32 | 33.12 | 1.78 | 0.43 | 5.53 | 3.82 | 20.59 ± 1.36 |

| | | | | | | | |
|---|---|---|---|---|---|---|---|
| Notes: Statistical analysis is performed with NGF-HA-PRTM-rHDL as control, * P < 0.05, ** P < 0.01, or *** P < 0.001 are significantly different from the NGF-HA-PRTM-rHDL group. | | | | | | | |

According to experimental results shown in Fig. 6, the NGF-carried nanocomplexes rapidly and efficiently promoted the differentiation of PC12 cells into neurons, with a significant increase in neuronal branching points and synaptic lengths having a morphology similar to that of neurons.

Fig. 6 shows that a nerve growth factor (NGF)-carried nanocomplexs promote PC12 cell differentiation. Fig. 6A shows neurite length statistics for different formulations promoting PC12 cell differentiation; Fig. 6B shows neurite branch point statistics for different formulations promoting PC12 cell differentiation; Fig. 6C is a morphogram of different formulations promoting PC12 cell differentiation. A recombinant lipoprotein nanocomplex (NGF -HA-PRTM-rHDL) carried with hyaluronic acid, protamine and NGF were co-incubated with PC12 cells for 8 h; the control was free NGF; an NGF-carried recombinant lipoprotein nanocomplex (NGF-rHDL) without hyaluronic acid and protamine; a blank liposome (liposome) and a culture solution blank control (Control).

### Example 6 Evaluation of intracellular enzymatic activity of nanocomplex carried with HRP

HRP was selected as a model for the study of active enzyme-based protein drugs, and the same preparation method as in Example 1 was used to prepare the experimental group of HRP-carried nanocomplexes and the control group, respectively, and the formulations of the experimental and control groups were shown in Table 9.

Hela cells were selected and inoculated in a 96-well plate. HRP was administered at a concentration of 10 ug/mL, and the cells were incubated with different protein-loaded formulations for 6 h. Then the cells were rinsed three times with PBS, 200 ul of TMB color development solution was added to each well. The same was incubated at room temperature and protected from light for 3-30 min until the color development reached the expected shade. The absorbance value was measured at 650 nm, which indicates the level of intracellular HRP enzyme activity.

According to the experimental results shown in Table 9, the intracellular horseradish peroxidase level is lowest in the group given free HRP, and the liposomes carried with hyaluronic acid, protamine, and HRP may partially deliver HRP enzyme to the cells, thereby increasing intracellular enzyme activity, while the recombinant lipoprotein nanocomplex (HRP-HA-PRTM-rHDL) carried with hyaluronic acid, fish sperm protein, and HRP significantly increases the intracellular horseradish peroxidase activity.

**Table 9 Characterization of HRP-carried nanocomplexes in different formulations and detection of intracellular enzyme activity**

| Formulation composition | DMPC (%) | DOPA (%) | HRP (%) | HA (%) | PRTM (%) | ApoE (%) | Size (nm) | Zeta potential (mV) | HRP-catalyzed TMB activity |
|---|---|---|---|---|---|---|---|---|---|
| HRP | 0 | 0 | 100 | 0 | 0 | 0 | Not detected | Not detected | 0.08 ± 0.01 (***) |
| HRP-HA-PRTM | 0 | 0 | 94.811 | 0.749 | 4.44 | J | 540.1 ± 59.95 | 15.17 ± 0.5 | 0.09 ± 0.006 (***) |
| HRP-rHDL | 46.06 | 31.41 | 17.9 | 0 | 0 | 4.63 | 35.07 ± 8.77 | -34.47 ± 5.36 | 0.07 ± 0.002 (***) |
| HRP-HA-PRTM-LIPO | 47.24 | 32.95 | 18.77 | 0.15 | 0.89 | 0 | 50.57 ± 28.4 | -41.5 ± 4.36 | 0.17 ± 0.15(**) |
| HRP-HA-PRTM-rHDL | 45.61 | 31.11 | 17.73 | 0.14 | 0.83 | 4.58 | 37.22 ± 7.28 | -48.87 ± 1.95 | 0.35 ± 0.02 |

| | | | | | | | | | |
|---|---|---|---|---|---|---|---|---|---|
| Notes: (a) "Not detected" means relevant detection is not made. (b) Statistical analysis is performed with HRP-HA-PRTM-rHDL as control. ** P<0.01 and *** P<0.001 are significantly different from the HRP-HA-PRTM-rHDL group. | | | | | | | | | |

### Example 7 Preparation, characterization and intracellular protein delivery of nanocomplex carried with catalase (CAT)

### (1) Preparation

(1.1) The catalase (CAT) was incubated with hyaluronic acid and protamine at different mass ratios to form a protein-carried complex (FITC-CAT-HA-PRTM).
(1.2) Preparation of liposomes by membrane hydration: the lipids (a combination of neutral phospholipid DMPC and anionic phospholipid DOPA) were weighed and placed in a 500 mL round-bottom flask. The mixture was added with 2 mL diethyl ether, dried to remove the water in the phospholipid, and added with 2 ml chloroform solution. The same was placed on a rotary evaporator, and vacuumized for 1 hour. 4 mL of 0.01 M PBS solution (pH 7.4) was added, and the mixture was shaken intermittently for 10 min in a 40°C water bath until the membrane was hydrated and exfoliated to obtain liposomes. The particle size of the liposomes was further reduced by ultrasound using a probe to obtain liposomes.
(1.3) The above prepared liposomes were co-incubated with FITC-CAT-HA-PRTM in different ratios to form liposomes carried with hyaluronic acid, protamine and FITC-CAT (FITC-CAT-HA-PRTM-LIPO); ApoE was added to the above liposome solution; the mixture was gently mixed and incubated on a shaker at 120 rpm, 37°C for 36 hours to obtain recombinant lipoprotein nanocomplexes carried with catalase CAT (FITC-CAT-HA-PRTM-rHDL).

### (2) Characterization

The catalase-carried nanocomplexes were negative stained with phosphotungstic acid, and the morphology was observed by a transmission electron microscopy. After further sample preparation, the structure was observed by the cryoelectroscope. The particle size and surface potential were measured by a laser granulometry. The encapsulating efficiency and drug carrying capacity of the catalase-carried nanocomplexes to the protein drugs were determined by a microplate reader.

### (3) Evaluation of intracellular protein delivery

The intracellular protein delivery of the catalase-carried nanocomplex was observed by a laser confocal microscopy. Microglia cells BV2 were inoculated in confocal dishes at a density of 50,000/well and cultured for 24 hours. The original culture solution was drawn and discarded, 500 µL of recombinant lipoprotein nanocomplex (FITC-CAT-HA-PRTM-rHDL) carried with green fluorescent protein labelled catalase (FITC-CAT) was added, and the free FITC-CAT proteins in the control group, respectively; a hyaluronic acid, protamine, FITC-CAT complex (FITC-CAT-HA-PRTM); fitc-CC-rHDL without hyaluronic acid and protamine; liposomes (FITC-CAT-HA-PRTM-LIPO) carried with hyaluronic acid, protamine, and FITC-CAT and FITC-CC + commercial protein transfection reagent (FITC-CAT-Pulsin) (administration concentration: 20 µg/mL, calculated according to the amount of FITC-CAT protein) were incubated at 37°C for 6 hours. Then it was fixed by 3.7% formaldehyde for 10 min at 37°C, stained for 10 min by Hoechest, and washed for 3 times by PBS. Confocal photography and qualitative observation were performed, and the Image J software was used for semi-quantitative analysis of FITC-CAT uptake.

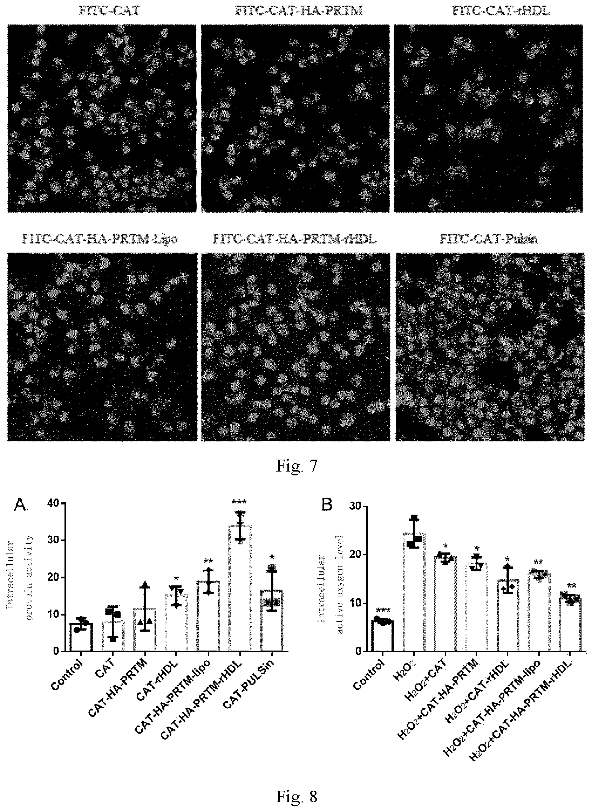

The results show that the particle size of FITC-CAT-HA-PRTM-rHDL in a proper proportion is below 100 nm, while the FITC-CAT-HA-PRTM had a large particle size (530.90±311.00 nm) further aggregates with time (standing overnight at 4°C), resulting in serious precipitation. When the particle size is detected, the detection instrument indicates that it does not meet the detection requirements and cannot be effectively detected. Compared to the FITC-CAT, FITC-CAT-HA-PRTM, FITC-CAT-rHDL, FITC-CAT-HA-PRTM-LIPO and commercial protein transfection formulation group, the optimized FITC-CAT-HA-PRTM-rHDL formulation delivers CAT more efficiently into cells, with the same more evenly distributed in the cytoplasm (Table 10, Fig. 7).

**Table 10 Characterization of nanocomplex carried with peroxidase in different formulations and quantitative data of microglia BV2 for uptaking proteins**

| Formulation composition | DMPC (%) | DOPA (%) | FITC-CAT (%) | HA (%) | PRTM (%) | ApoE (%) | Size (nm) | Zeta potential (mV) | Encapsulati on rate (%) | Cell uptake mean optical density value |
|---|---|---|---|---|---|---|---|---|---|---|
| FITC-CAT | 0 | 0 | 100 | 0 | 0 | 0 | Not detected | Not detected | Not detected | 1.88±0.04*** |
| FITC-CAT-HA-PRTM | 0 | 0 | 84.7 | 1.4 | 13.9 | 0 | 530.90 ± 311.00 | -1.31 ± 1.47 | 86.80 ± 1.24 | 2.72±0.19*** |
| FITC-CAT-rHDL | 36.2 | 26.3 | 32.7 | 0 | 0 | 4.8 | Not detected | Not detected | 53.81 ± 3.23 | 2.58±0.04*** |
| FITC-CAT-HA-PRTM-LIPO | 35.8 | 26.0 | 32.4 | 0.5 | 5.3 | 0 | 69.71 ± 14.74 | -20.20 ± 3.82 | 79.33 ± 2.68 | 2.72±0.18*** |
| FITC-CAT-HA-PRTM-rHDL | 34.2 | 24.8 | 30.9 | 0.5 | 5.1 | 4.5 | 63.62 ± 1.97 | -19.43 ± 1.96 | 68.66 ± 4.72 | ± 0.16 |

| | | | | | | | | | | |
|---|---|---|---|---|---|---|---|---|---|---|
| Notes: (a) "Not detected" means relevant detection is not made. (b) Statistical analysis is performed with FITC-CAT-HA-PRTM-rHDL as control. *** P < 0.001 is significantly different from FITC-CAT-HA-PRTM-rHDL. | | | | | | | | | | |

Fig. 7 shows a fluorescence distribution diagram of BV2 cells for uptaking different formulations of catalase. Green fluorescent protein labelled cytochrome C (FITC-CAT); a recombinant lipoprotein nanocomplex (FITC-CAT-HA-PRTM-rHDL) carried with a hyaluronic acid, protamine, and FITC-CAT complex; a hyaluronic acid, protamine, and FITC-CAT complex (FITC-CAT-rHDL); a FITC-CAT-carried recombinant lipoprotein nanocomplex (FITC-CAT-HA-PRTM-LIPO) without hyaluronic acid and protamine; a liposome (FITC-CAT-HA-PRTM- LIPO) carried with hyaluronic acid, protamine, and FITC-CAT + commercial protein transfection reagent (FITC-CAT-Pulsin).

### Example 8 Evaluation of transfection capacity, antioxidant capacity and cell viability of catalase (CAT)-carried nanocomplex on HT22 cells

Catalase (CAT) was selected as a model for the study of protein drugs, and the same preparation method as in Example 7 was used to prepare the nanocomplexes carried with oxidase (CAT), respectively, and the formulations were shown in Table 11.

The transfection ability of CAT-carried nanocomplexs on HT22 cells was detected by a catalase assay kit. The reaction principle is that the catalase can catalyze hydrogen peroxide to produce water and oxygen when hydrogen peroxide is relatively sufficient. The residual hydrogen peroxide, catalyzed by peroxidase, can oxidize the chromogenic substrate to produce a red product with an absorption maximum at 520 nm. A standard curve is made by using the hydrogen peroxide standard, so as to calculate how much amount of hydrogen peroxide catalyzed by the catalase in the sample is converted into water and oxygen per unit volume and unit time, and thus calculate the enzyme activity of the catalase in the sample. According to the specific experimental protocol, HT22 cells were inoculated in 12-well plates, cultured in a carbon dioxide incubator for 24 h, then administered with different formulations loaded with CAT protein, including a recombinant lipoprotein nanocomplex (CAT-HA-PRTM-rHDL) containing hyaluronic acid, protamine and CAT and control groups (Control) being an untreated group (Control), a free CAT protein group, CAT + commercial protein transfection reagent group (CAT-Pulsin) (administration concentration is 20 µg/mL, calculated according to the amount of CAT protein), which were incubated at 37°C for 6 h. Cells were lysed and intracellular catalase levels were measured according to the manufacturer's instructions.

According to results shown in Fig. 8A, the untreated Control group and the group administered with free CAT has the lowest intracellular catalase levels. The commercial protein transfection reagent Pulsin may partially deliver CAT protein into cells to increase intracellular enzyme activity, while the recombinant lipoprotein nanocomplex containing hyaluronic acid, protamine and CAT (CAT-HA-PRTM-rHDL) significantly increases the intracellular catalase activity.

**Table 11 Catalase-carried nanocomplex in different formulations increase catalase activity in microglial BV2 cells**

| Formulation composition | DMPC (%) | DOPA (%) | CAT (%) | HA (%) | PRTM (%) | ApoE (%) | Enzyme activity level |
|---|---|---|---|---|---|---|---|
| Formulation 1 | 34.8 | 17.3 | 22.39 | 0.12 | 21.08 | 4.31 | 6.12 ± 4.06 |
| Formulation 2 | 27.33 | 13.31 | 39.4 | 16.6 | 0.43 | 2.93 | 10.60 ± 2.53 (ns) |
| Formulation 3 | 39.91 | 37.22 | 12.06 | 1.53 | 4.85 | 4.43 | 15.17 ± 3.04 (*) |
| Formulation 4 | 34.2 | 24.8 | 30.9 | 0.5 | 5.1 | 4.5 | 33.97 ± 3.65 (***) |

| | | | | | | | |
|---|---|---|---|---|---|---|---|
| Notes: Formulation 1 is used as a control. * P < 0.05 or * * * P < 0.001 is significantly different from Formulation 1. ns means that there is no significant difference in the level of enzyme activity between Formulation 2 and Formulation 1. | | | | | | | |

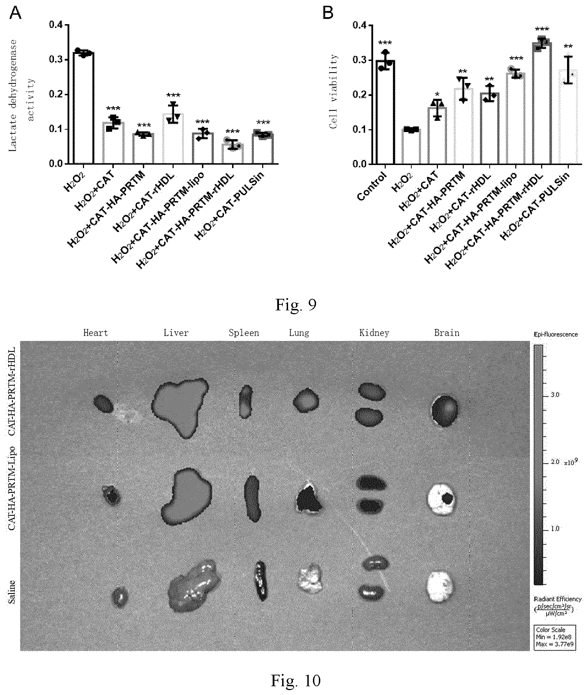

Since CAT protein has antioxidant function, hydrogen peroxide is used to induce the increase of intracellular oxidative stress level to evaluate the regulatory effect of CAT-carried nanocomplexs on the intracellular oxidative stress level. The specific experimental method is as follows. HT22 cells were inoculated in a 96-well plate, and different pharmaceutical formulations loading CAT protein were administered when the confluence reached 70%. After administration for 4 hours, the formulation solution was discarded. The fluorescent probe DCFH-DA was added to incubate with the cells for 30 min, and then the probe solution was discarded. 400 µM hydrogen peroxide was added to incubate with the cells for 15 min to induce the oxidative damage, and the quantitative analysis was performed by a microplate reader. According to the results shown in Fig. 10, the Control group shows the intracellular reactive oxygen level in the completely untreated group, the intracellular ROS levels are increased dramatically after the induction of dioxidation alone, while the administration of CAT-HA-PRTM-rHDL significantly reduces the increase of cellular ROS levels induced by hydrogen peroxide.

Fig. 8 shows that CAT-HA-PRTM-rHDL effectively increases intracellular catalase levels (Fig. 8A) and effectively reduces H₂O₂-induced increase in intracellular ROS levels (Fig. 8B).

The lactate dehydrogenase cytotoxicity assay kit mainly evaluates cell membrane integrity, and is also one of the indicators for evaluating cell status. The cell administration step is the same as above. After 4 hours of administration, the formulation solution was discarded, and hydrogen peroxide was added to incubate with the cells to induce their oxidative damage. At 1 hour before the scheduled detection time point, the cell culture plate was taken out from the cell incubator, and the LDH release reagent provided by the kit was added in an amount of 10% of the original culture solution volume into the "control well for maximum enzyme activity of sample". After adding LDH release reagent, the mixture was repeatedly blown and hit for several times and mixed well, and then continued to incubate in the cell incubator. After reaching the preset time, use the cell culture plate was centrifuged by a multi-well plate for 5 min at 400g. 120 µl supernatant was respectively taken from each well and added into a corresponding well of a new 96-well plate so as to make sample determination. The operation steps of CCK8 were basically the same as above. Firstly, different protein formulations were added to incubate with cells for 4h, then the formulation solution was discarded, and hydrogen peroxide was added to incubate with cells to induce their oxidative damage. CCK8 reagent was added to detect cell viability at a predetermined detection time point. According to experimental results shown in Fig. 9, increasing cellular ROS levels significantly reduces the membrane damage induced by hydrogen peroxide and increases the levels of cell viability.

Fig. 9 shows that CAT-HA-PRTM-rHDL effectively inhibites membrane damage induced by H₂O₂ (Fig. 9A) and effectively increases cell viability (Fig. 9B).

### Example 9 Evaluation of the in vivo and intracerebral distribution of catalase (CAT)-loaded nanocomplex in mice

Fluorescence probes DiR and DiI labelled recombinant lipoprotein nanocomplexes (CAT-HA-PRTM-rHDL) (i.e., experimental group FITC-CAT-HA-PRTM-rHDL of Example 7) carried with hyaluronic acid, protamine, CAT and liposomes (CAT-HA-PRTM-LIPO) carried with hyaluronic acid, protamine, and CAT were prepared. Meanwhile, a mouse model of controlled cortical injury (CCI) was established. The mice were anesthetized by intraperitoneal injection of 5% chloral hydrate and fixed in a stereotaxic apparatus. The scalp was incised under aseptic conditions to expose the right parietal bone. A round cranial window was opened between the right coronary suture and the herringbone suture, and the dura mater was exposed. The cortical injury model was simulated by setting different hit parameters. Hit parameters: velocity 1.5 m/s, depth 1 mm, head hit diameter 2 mm, and contact time 100 ms. A moderate injury condition was simulated for conventional brain injury experiments. After injury, the skull was closed and the skin sutured. In the sham-injured Sham group, only the scalp was incised to expose the right parietal bone without hit injury. For the in vivo distribution experiment of the formulation, the DiR-labelled formulation was administered via the tail vein (calculated according to the concentration of phospholipid DMPC of 20 mg/kg). The heart, liver, spleen, lung, kidney and brain tissues were taken out 3 hours after administration, washed with normal saline, and then placed in a small animal living imager to collect images, so as to observe the distribution of nanocomplex in the body and its dynamic change of transport to the brain. According to the experimental results shown in Fig. 10, no fluorescent agent is distributed in the brain of the saline-administered group, while CAT-HA-PRTM-rHDL has a significant fluorescence intensity in the brain and is highly concentrated at the CCI lesion site of the right brain, indicating that the nanocomplex constructed in the present invention can efficiently deliver the protein of interest into the brain and target the lesion site.

Fig. 10 shows that in the cortical injury model of C57 mice, the fluorescent dye DiR-labelled recombinant lipoprotein nanocomplex (CAT-HA-PRTM-rHDL) carrying hyaluronic acid, protamine and CAT and liposome (CAT-HA-PRTM-LIPO) carrying hyaluronic acid, protamine and CAT are administered via the tail vein. Four hours after administration, the organs of mice are taken for living imaging to evaluate the in vivo distribution and brain penetration of the formulation.

### Example 10 Evaluation of the intracerebral distribution of catalase (CAT)-carried nanocomplex (i.e., the experimental group FITC-CAT-HA-PRTM-rHD of Example 7) in mice

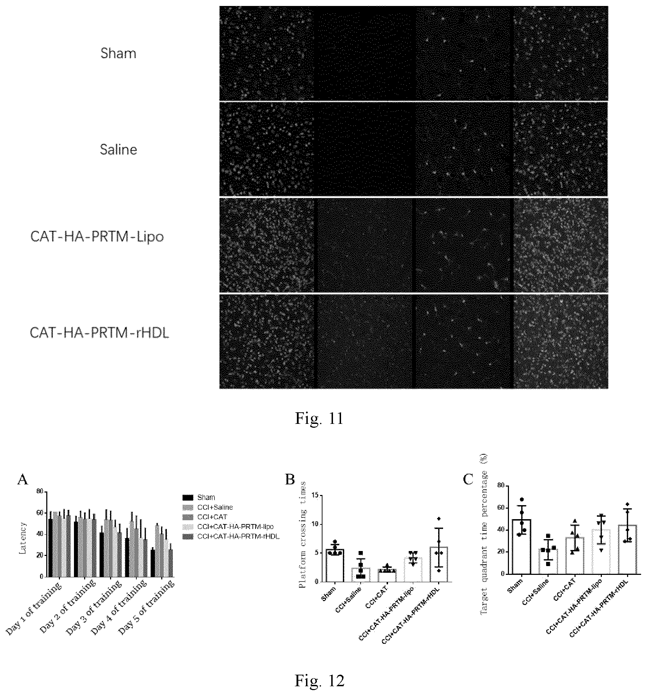

Furthermore, according to the present invention, CX3CR1-GFP transgenic mice with microglia cells transformed into the green fluorescent protein were selected to construct a CCI model. DiI-labelled fluorescent formulation was also administered into the tail vein after CCI. The distribution of DiI-CAT-HA-PRTM-rHDL formulation in the brain of CCI mice and the uptake thereof by microglia cells were evaluated through a brain slice experiment. After the administration into the tail vein for 3 hours, the mice were anesthetized and fixed for cardiac perfusion, and the whole brain of tumor-bearing mice was taken and fixed in 4% paraformaldehyde for 24 hours. After rinsing with PBS, the same was placed in 15% and 30% sucrose solution successively to dehydrate until they sunk, then embeded by using O. C. T. and frozen at -20°C, with continuous frozen coronal section made, and the section thickness of 14 µm. After rinsing with PBS, the same was stained for 10 min with 100 ng/mL DAPI, rinsed with PBS, wiped to remove the water stain. The section was sealed with glycerol phosphate, and the intracerebral distribution of CAT-HA-PRTM-rHDL was observed under the laser confocal microscope. According to the experimental results shown in Fig. 11, CAT-HA-PRTM-rHDL can be efficiently uptaken by microglia in the brain.

Fig. 11 shows the establishment of a cortical injury model in CX3CR1-GFP mice by administering different formulations labelled with the fluorescent dye DiI via the tail vein. Four hours after administration, mice brains are taken for cryosection to evaluate the uptake of CAT-carried nanocomplexs by microglia in the brain in vivo.

### Example 11 Effect of catalase (CAT)-loaded nanocomplex (i.e., the experimental group FITC-CAT-HA-PRTM-rHDL of Example 7) on spatial learning and memory capacity in CCI mice

The model of moderate CCI brain damage in mice was established. Different formulations loaded with CAT (the dose concentration was 13,300 Units/kg according to CAT calculation) were administered via the tail vein. The mice were trained and tested for the behavior using Morris water maze after continuous administration for 7 days. Water maze consisted of a circular pool, a platform and a recording system. The pool had a diameter of 150 cm and a height of 50 cm. The pool was divided into four quadrants (quadrants I, II, III and IV). The pool was filled with water to a depth of 30 cm. White food color was added to make the water opaque white, so that the experimental mice could not directly see the platform and the bottom of the pool. The water temperature was kept around 25°C. A spatial reference (door, camera, wall sign, etc.) was placed around the pool and the position was kept constant for mouse positioning and memorizing the position of the platform. The cylindrical platform, 9 cm in diameter and 29 cm high, was wrapped in non-reflective black cloth and placed in the quadrant IV with the plane 1 cm below the water surface. A camera was placed above the center of the pool, and animal swimming images were automatically collected. The collected signals were directly input into a computer, and the mouse swimming trajectory was monitored and recorded using the Morris water maze video analysis system 2.0. Hidden platform test began 7 days after continuous dosing of mice, and continued for 5 days. The water entry point of each training was 4 quadrants arranged according to the random principle. Each time different mice are placed into the water facing the pool wall at the same position. The order of water entry was different every two adjacent days. The computer monitored and recorded the route from the water entry, beginning to find, finding and climbing the black platform and the required time (latency). Each mouse was trained 4 times a day, and each training was set to have a latency period of 60 s. If no platform was found within 60 s, it was necessary to lead the mouse to the platform and let it stay for 10 s. At this time, the latency period was recorded as 60 s, and each mouse had a training interval of 30 s every two times. Spatial Exploratory Experiment (Probe trial): After the 5-day positional navigation test, the platform was removed on the 6th day, and the mice were placed into the water facing the pool wall from the water entry points in the quadrants II and III, respectively. The percentage of time the mice spent in the target quadrant (the quadrant in which the platform was located) within 60 s and the trajectory of mice searching for the platform were recorded. As shown in Fig. 12, as the training time increased, the latency of mice became shorter, and CAT-HA-PRTM-rHDL significantly shortened the latency of mice to find the platform. Meanwhile, the spatial exploratory experiments showed that the number of platform crossing and the residence time on the target platform were significantly increased in mice given CAT-HA-PRTM-rHDL, suggesting that CAT-HA-PRTM-rHDL efficiently delivered CAT protein to the brain injury site to alleviate the increased level of oxidative stress caused by brain injury and improve the spatial learning and memory ability of mice.

Fig. 12 shows the establishment of a cortical injury model in C57 mice and a Sham group being a sham injury control group, with administration continued for 1 week after modeling. The effects of different formulatons loading CAT on the latency, the times of crossing the platform and the time in the target quadrant of CCI model mice are investigated by the Morris water maze test.

### Example 12 Effect of catalase (CAT)-loaded nanocomplex (i.e., the experimental group FITC-CAT-HA-PRTM-rHDL of Example 7) on exercise ability and survival time of SOD mice

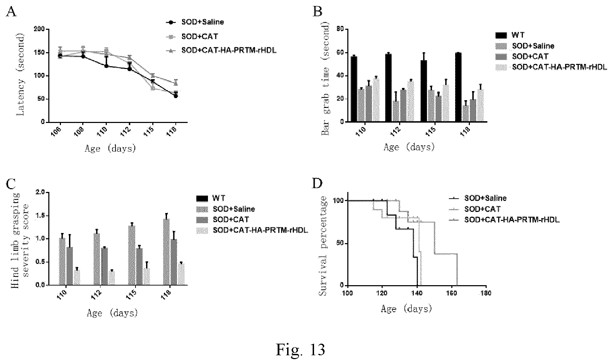

Transgenic mice were administered with SOD 1-G93A (Amyotrophic Lateral Sclerosis Model) CAT-HA-PRTM-rHDL as in Example 10, in which Saline and free CAT protein were administered as a control group, and SOD mice were administered starting at 3 months of age and continuing daily for exercise capacity evaluation, including rotarod test, grab bar test and hind limb grasping test. The specific steps of the stick-turning test were as follows. The mice were trained three times in the first week, and the stick-turning speed was 14 rpm. At this turning speed, the mice staying on the turning stick for 180 seconds was defined as asymptomatic. When the mice staying on the turning stick for less than 180 seconds was defined as clinical onset time, and the mice can be trained for one week before the test experiment. According to the grab bar test, the time that the mouse depended on the upper limb grasping bar, the total detection time was 60 seconds, and the falling latency of the mouse within 60 seconds was recorded. According to the hind limb grasping test, the mouse was hung upside down by grasping the tail of mouse, the video was recorded for 15 seconds and the grasping of mouse hind limb within 15 seconds was recorded. The score of 0-3 was used to evaluate the grasping degree of mouse hind limb (proportional to the severity of stiffness). According to the results shown in Fig. 13, in rotarod test, the retention time on the rotarod was similar in each group of mice at the initial stage of the test. With the extension of time (storing overnight at 4°C), the retention time on the rotarod was significantly prolonged in the mice given CAT-HA-PRTM-rHDL, while in the hanging wire duration test, the hanging wire time was significantly prolonged in the mice given CAT-HA-PRTM-rHDL group compared with the control group. The decreased clasping behavior of hind limbs indicated that CAT-HA-PRTM-rHDL alleviated the pathological process of paralysis in the SOD mice.

Fig. 13 shows the use of the SOD mouse model, with administration continued. The effects of different formulations loaded with CAT on the exercise ability of SOD transgenic mice are also evaluated in the rotarod test, the hanging wire duration and the hind-limb clasping behavior test.

### Example 13 Preparation, characterization and cellular uptake efficiency and intracerebral distribution of protein-free blank nanocomplex

### (1) Preparation

(1.1) HA-PRTM was formed by co-incubation of hyaluronic acid and protamine at different mass ratios.
(1.2) Preparation of liposomes by membrane hydration: the lipids (neutral phospholipid DMPC and anionic phospholipid DOPA) and red fluorescent dye DiI were weighed and placed in a 500 mL round-bottom flask. The mixture was added with 2 mL diethyl ether, dried to remove the water in the phospholipid, and added with 2 ml chloroform solution. The same was placed on a rotary evaporator, and vacuumized for 1 hour. 4 mL of 0.01 M PBS solution (pH 7.4) was added, and the mixture was shaken intermittently for 10 min in a 40°C water bath until the membrane was hydrated and exfoliated to obtain liposomes. The particle size of the liposomes was further reduced by sonication with a sonicator probe to obtain a liposome carried with a red fluorescent probe (DiI-LIPO).
(1.3) The liposomes were incubated with HA-PRTM at different ratios to form blank liposomes that do not contain protein drugs (DiI-HA-PRTM-LIPO). Apolipoprotein (ApoE) was added into the above liposome solution. The mixture was gently mixed well, and incubated on a shaker at 120 rpm at 37°C for 36 hours to obtain a protein-drug-free nanocomplex (DiI-HA-PRTM -rHDL).

### (2) Characterization

The nanocomplexes without protein drugs were negative stained with phosphotungstic acid, and the morphology was observed by a transmission electron microscopy. After further sample preparation, the structure is observed by the cryoelectroscope. The particle size and surface potential were measured by a laser granulometry.

### (3) Cell uptake efficiency evaluation

Cellular uptake of the protein drug-free nanocomplexs was observed by the laser confocal microscopy. Human cervical cancer cells, Hela cells, were inoculated at a density of 50,000 cells/well in a confocal dish and cultured for 24 hours. The original culture solution was drawn and discarded, and 500 µL of the protein-drug-free nanocomplex labelled with a red fluorescent probe was added.

Experimental group: protein-drug-free nanocomplex (DiI-HA-PRTM-rHDL) labelled with red fluorescent probe. Control group: The nanocomplex (DiI-BSA-HA-PRTM-rHDL) loaded with a red fluorescent probe-labelled BSA protein drug was prepared as in Example 1. The experimental group and the control group were incubated with cells at 37°C for 4 hours (administration concentration: 10 µg/mL, calculated according to the mass of phospholipid DMPC), respectively. The cells were fixed with 3.7% formaldehyde for 10 min at 37°C, nuclei-stained with Hoechst for 10 min, and washed for 3 times by PBS. Confocal photography and qualitative observation were performed, and the Image J software was used for semi-quantitative analysis of cellular uptake efficiency.

### (3) Evaluation of intracerebral distribution of nanocomplex

C57 mice were used to construct a CCI model, and DiI-labelled formulations were injected through the tail vein after CCI. The distribution of DiI-BSA-HA-PRTM-rHDL and DiI-HA-PRTM-rHDL formulations in the brain of CCI model mice were evaluated by brain section analysis. At 3 h post tail intravenous injection, the mice were anaesthetized and heart perfused with saline and 4% paraformaldehyde. Then the brains were fixed in 4% paraformaldehyde, dehydrated in 15%, 30% sucrose solution, imbedded in OCT and sectioned at 14 µm. After rinsing with PBS, the section was stained for 10 min with 100 ng/mL DAPI, rinsed with PBS, wiped to remove the water stain. The section was sealed with glycerol phosphate, and the intracerebral distribution of two formulations was observed under a confocal microscope.

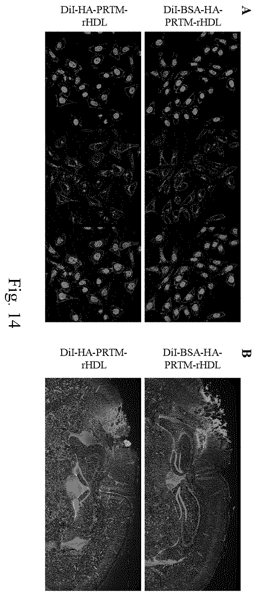

Fig. 14 shows the establishment of a cortical injury model in C57 mice by administering different formulations labeled with the fluorescent dye DiI via the tail vein. Four hours after administration, mice brains are taken for cryosection to evaluate the distribution of protein-carried or non-carried nanocomplexs in mice brains.

According to the results shown in Fig. 14, the particle sizes of the nanocomplex containing BSA protein drugs (DiI-BSA-HA-PRTM-rHDL) and blank liposomes that do not contain protein drugs (DiI-HA-PRTM-rHDL) were below 100 nm, which was beneficial for the formulation to penetrate the biological membrane barrier in vivo, and could efficiently improve the uptaking by cells (Fig. 14A). Meanwhile, in the mouse cortical injury model, both protein-free and protein-containing nanocomplexs efficiently targeted the brain injury site (Fig. 14B). These findings suggest that neither the protein carrying nor non-protein carrying does affect cellular uptake and targeted distribution in the brain, and that the carrier itself and the protein carrying formulation have similar cellular uptake and in vivo distribution behaviors.

**Table 12 Characterization of protein and protein-free nanocomplexs with different formulations**

| Formulation composition | DMPC (%) | DOPA (%) | FITC-BSA (%) | HA (%) | PRTM (%) | ApoE (%) | Size (nm) | Zeta potential (mV) | Cell uptake mean optical density value |
|---|---|---|---|---|---|---|---|---|---|
| Dil-BSA-HA-PRTM-rHDL | 37 | 26 | 30 | 0.35 | 2 | 4.65 | 39.48 ± 1.81 | -45.67 ± 3.50 | 5.42 ± 0.16 |
| DiI-HA-PRTM-rHDL | 52.17 | 37.72 | 0 | 0.33 | 3.26 | 6.52 | 36.30 ± 6.41 | -52.07 ± 2.15 | 6.1 ± 0.26 |

While particular embodiments of the present invention have been described, it will be understood by those skilled in the art that these are merely illustrative and that various changes and modifications may be made for the embodiments without departing from the principles and spirit of the invention. Accordingly, the protection sought herein is as set forth in the claims below.

## Claims

1. A nanocomplex, wherein the nanocomplex comprises 0-60% of protein drug, 0.03-15% of hyaluronic acid, 0.1-20% of protamine, 35-95% of lipid component and 2.5-40% of "apolipoprotein and/or a mimetic peptide thereof; the lipid component comprises an electrically neutral lipid and an anionic lipid; the total amount of the hyaluronic acid and the protamine is 0.03-15%; the above percentages are the respective mass percentages of the components relative to the nanocomplex.

2. The nanocomplex according to claim 1, wherein the nanocomplex does not comprise cationic lipid DOPAT;
and/or, the molecular weight of the protein drug is 10-255 kDa, for example, 10-16 kDa, 15-40 kDa, 30-50 kDa, 60-80 kDa 140-180 kDa, 200-255 kDa or 210-255 kDa, preferably 10-14 kDa, 20-33 kDa, 35-45 kDa, 60-80 kDa, 150-170 kDa, 220-250 kDa, 220-250 kDa, for another example, 12.4 kDa, 26 kDa, 40 kDa, 69.3 kDa, 160 kDa or 240 kDa;
and/or, the isoelectric point of the protein drug is 4-11, for example, 4-5.3, 3.7-5.7, 4.4-6.4, 6-8.5, 7-9, 8.3-10.3 or 9.3-11, preferably 4-4.8, 4.2-5.5, 4.9-5.9, 6.5-8, 7.5-8.5, 8.8-9.8 or 9.8-10.8, for another example, 4.3, 4.7, 5.4, 7.2, 8, 9.3 or 10.3;
and/or, the electrically neutral lipid comprises one or more of an amphipathic lipid, a nonionic lipid, cholesterol and derivatives thereof, preferably comprises one or more of phosphatidyl choline, phosphatidyl ethanolamine, phosphatidylglycerol and sphingomyelin;
the phosphatidylcholine is preferably one or more of dimyristoyl phosphatidylcholine DMPC, dipalmitoyl phosphatidylcholine DPPC, distearoyl phosphatidylcholine DSPC, dioleoyl phosphatidylcholine DOPC, dilaroyl phosphatidylcholine DLPC, dierucoyl phosphatidylcholine DEPC, 1-palmitoyl-2-oleoyl phosphatidylcholine POPC, egg yolk lecithin, soybean phospholipid, hydrogenated soybean phosphatidylcholine HSPC and derivatives thereof, more preferably, one or more of dimyristoyl phosphatidylcholine DMPC, egg yolk lecithin and hydrogenated soybean phosphatidylcholine HSPC;
the phosphatidyl ethanolamine is preferably one or more of dimyristoyl phosphatidyl ethanolamine DMPE, distearoyl phosphatidyl ethanolamine DSPE, dipalmitoyl phosphatidyl ethanolamine DPPE, dioleoyl phosphatidyl ethanolamine DOPE, distearoyl phosphatidyl ethanolamine-polyethylene glycol 2000, distearoyl phosphatidyl ethanolamine-polyethylene glycol 5000, dipalmitoyl phosphatidyl ethanolamine-polyethylene glycol 2000, dipalmitoyl phosphatidyl ethanolamine-polyethylene glycol 5000 and derivatives thereof;
the phosphatidylglycerol is preferably one or more of dimyristoyl phosphatidylglycerol DMPG, distearoyl phosphatidylglycerol DSPG, dipalmitoyl phosphatidylglycerol DPPG, dioleoyl phosphatidylglycerol DOPG, 1-palmitoyl-2-oleoyl phosphatidylglycerol POPG-Na, egg yolk phosphatidylglycerol EPG and derivatives thereof;
preferably, when the electrically neutral lipid is a mixture of dimyristoyl phosphatidylcholine DMPC and egg yolk lecithin, the mass ratio of dimyristoyl phosphatidylcholine DMPC to egg yolk lecithin may be 20:(8-12), for example, 20:10.94;
and/or, the anionic lipid is an anionic phospholipid; the anionic phospholipid preferably comprises one or more of phosphatidic acid, phosphatidylinositol, phosphatidylserine, cardiolipin, lysophospholipin and ganglioside, more preferably phosphatidic acid and/or ganglioside;
wherein the phosphatidic acid is preferably one or more of dimyristyl phosphatidic acid DMPA, distearoyl phosphatidic acid DSPA, dipalmitoyl phosphatidic acid DPPA, dioleoyl phosphatidic acid DOPA and derivatives thereof, more preferably, dioleoyl phosphatidic acid DOPA;
the phosphatidylserine is preferably dioleoyl phosphatidylserine DOPS and/or dipalmitoyl phosphatidylserine DPPS;
the lysophospholipid is preferably one or more of stearoyl lysolecithin S-lysoPC, myristoyl lysolecithin M-LysoPC, palmitoyl lysolecithin P-LysoPC and derivatives thereof;
the ganglioside is preferably monosialotetrahexosylganglioside GM1;
preferably, when the anionic phospholipid is a mixture of phosphatidic acid and ganglioside, the mass ratio of ganglioside to phosphatidic acid is 30.07:(3-7), for example, 30.07:5;
and/or, the "apolipoprotein and/or the mimetic peptide thereof' is one or more of ApoE and a mimetic peptide thereof, ApoA-I, ApoA-II, ApoA-IV and a mimetic peptide thereof, ApoC-I, ApoC-II, ApoC-III and a mimetic peptide thereof, ApoB and a mimetic peptide thereof, ApoJ and a mimetic peptide thereof, for example, ApoE or a mimetic peptide of ApoA-I;
and/or, the amount of the protein drug is 1-43%, for example, 1.5%, 1.78%, 2.2%, 3%, 4%, 4.8%, 5%, 6%, 8%, 8.5%, 9%, 10%, 11%, 12.06%, 15%, 16%, 16.5%, 17%, 17.73%, 19%, 20%, 22%, 28.08%, 28.68%, 29.31%, 30%, 31.5%, 32%, 30.9%, 33%, 33.07%, 34%, 34.5% or 41%;
and/or, the amount of the hyaluronic acid is 0.03%-5%, for example, 0.04%, 0.05%, 0.06%, 0.08%, 0.09%, 0.1%, 0.13%, 0.14%, 0.15%, 0.2%, 0.24%, 0.25%, 0.3%, 0.33%, 0.35%, 0.41%, 0.43%, 0.44%, 0.45%, 0.47%, 0.5%, 0.6%, 0.78%, 0.8%, 0.9%, 1.53%, 2.1%, 0.2%, 2.1%, 2.5% or 3%;
and/or, the amount of the protamine is 0.2%-16%, for example, 0.1%, 0.2%, 0.3%, 0.35%, 0.8%, 0.83%, 1%, 1.3%, 1.5%, 1.6%, 1.7%, 2%, 2.34%, 2.39%, 2.44%, 3%, 3.26%, 3.3%, 3.5%, 3.7%, 4%, 4.3%, 4.5%, 4.85%, 5%, 5.1%, 5.5%, 5.53%, 6%, 6.5%, 7%, 13% or 14%;
and/or, the amount of the "apolipoprotein and/or the mimetic peptide thereof' is 2.85%-25%, for example, 2.92%, 3%, 3.5%, 3.82%, 3.94%, 4%, 4.1%, 4.12%, 4.43%, 4.5%, 4.65%, 4.68%, 4.78%, 4.83%, 4.89%, 5%, 5.4%, 5.8%, 6%, 6.35%, 6.52%, 7.21%, 10.35%, 16.5%, 16.37%, 19.29% or 20%;
and/or, the amount of the lipid component is 35%-90%;
and/or, the amount of the anionic lipid is 14%-40%, for example 16%, 17.64%, 22%, 24%, 24.04%, 24.78%, 24.8%, 25%, 25.66%, 26%, 26.1%, 27%, 27.22%, 27.5%, 29.79%, 29%, 30%, 30.5%, 30.66%, 31%, 31.08%, 31.11%, 32%, 31.5%, 32%, 32.5%, 33%, 33.06%, 33.12%, 33.6%, 34%, 34.44%, 35%, 35.07%, 36%, 37.22%, 37.72%, 37.8%, 38% or 39%;
and/or, the amount of the electrically neutral lipid is 22%-60%, for example, 23.5%, 24.36%, 30%, 30.94%, 32%, 33%, 34.2%, 34.22%, 35%, 36.09%, 37%, 37.44%, 37.5%, 38.25%, 39.08%, 39.91%, 41%, 41.21%, 42%, 42.34%, 42.92%, 43.5%, 44%, 45%, 45.61%, 46%, 46.4%, 47%, 47.56%, 48.43%, 50%, 52.17%, 52.2%, 53%, 54%, 54.5%, 55.32%, 56% or 57%;
or, the total amount of the hyaluronic acid and the protamine is 0.2%-14%, for example, 0.3%, 0.5%, 0.64%, 0.8%, 0.85%, 0.97%, 1%, 1.08%, 1.15%, 1.2%, 1.5%, 1.63%, 1.71%, 1.79%, 2%, 2.15%, 2.35%, 2.58%, 2.63%, 2.68%, 3.2%, 3.5%, 4%, 4.17%, 5%, 5.08%, 5.5%, 5.6%, 5.96%, 6.38%, 6.5%, 7%, 8%, 8.6%, 13.06% or 13.5%;
and/or, the particle size of the nanocomplex is 10-1000 nm, preferably 10-100 nm, for example 12-95 nm, further for example, 20.30±5.89 nm, 23.79±7.91 nm, 25 nm, 26.52±4.31 nm, 27.31±10.84 nm, 27.38±7.83 nm, 27.55±6.99 nm, 37.98±14.29 nm, 28.96±8.74 nm, 31.11±3.44 nm, 36.30±6.41 nm, 37.22±7.28 nm, 37.55±13.73 nm, 37.63±4.20 nm, 37.68±2.20 nm, 38 nm, 39.12±4.84 nm, 40.55±7.66 nm, 55 nm, 55.75±7.69 nm, 57 nm, 60 nm, 63.62±1.97 nm, 70 nm, 74.20±14.23 nm or 75.29 ± 14.53 nm;
and/or, the nanocomplex has a Zeta potential of -70 to -15 mV, for example, -65, - 64.87±3.30, -63.7±2.66, -58.87±4.90, -57.33±2.31, -56.33±3.26, -55.20±10.74, -52.07±2.15, - 50.10±3.18, -48.87±1.95, -45.20±2.15, -44.87±0.45 mV, -43.93±14.03, -43.87±9.68, - 43.20±2.75 mV, -40.23±6.92, -38.27±13.10, -36.83±2.71, -31.57±4.67, -30, -25, -20, - 21.03±2.47 or -19.43±1.96 mV

3. The nanocomplex according to claim 1 or 2, wherein when the isoelectric point of the protein drug is 4-5.7 and the molecular weight is 60-80 kDa, the nanocomplex comprises 0-30% of protein drug, 0.15-2.1% of hyaluronic acid, 1-6.5% of protamine, 4.65-7% of "apolipoprotein and/or the mimetic peptide thereof", 30-53.5% of electrically neutral phospholipid and 23.5-38.5% of anionic phospholipid; the total amount of the hyaluronic acid and the protamine is 1-9%;
the isoelectric point of the protein drug is preferably 4.2-5.5, for example, 4.7;
the molecular weight of the protein drug is preferably 60-80 kDa, for example, 69.3 kDa;
the protein drug is preferably bovine serum albumin;
the amount of the protein drug is preferably 0%, 9%, 16%, 22%, 28.08%, 28.68%, 29.31% or 30%;
the amount of the hyaluronic acid is preferably 0.15%, 0.24%, 0.33%, 0.35%, 0.47% or 2.1%;
the amount of the protamine is preferably 1%, 2%, 2.34%, 2.39%, 2.44%, 3.26%, 3.7% or 6.5%;
the amount of the "apolipoprotein and/or the mimetic peptide thereof" is preferably 4.65%, 4.68%, 4.78%, 4.83%, 4.89%, 5.4%, 6.35% or 6.52%;
the "apolipoprotein and/or the mimetic peptide thereof' is preferably ApoE;
the electrically neutral phospholipid is preferably DMPC and/or egg yolk lecithin, or DMPC and/or hydrogenated soybean phosphatidylcholine;
when the electrically neutral phospholipid is a mixture of DMPC and egg yolk lecithin, the mass ratio of DMPC to egg yolk lecithin is preferably 20:(8-12), for example 20:10.94;
the amount of the electrically neutral phospholipid is preferably 30.94%, 37%, 37.44%, 38.25%, 39.08%, 43.5%, 48.43% or 52.17%;
the anionic phospholipid is preferably ganglioside and/or phosphatidic acid; the phosphatidic acid is preferably dioleoyl phosphatidic acid DOPA; the ganglioside is preferably monosialotetrahexosylganglioside GM1;
when the anionic phospholipid is a mixture of monosialotetrahexosylganglioside GM1 and DOPA, the mass ratio of monosialotetrahexosylganglioside GM1 to DOPA is preferably 30.07:(3-7), for example, 30.07:5;
the amount of the anionic phospholipid is preferably 24.04%, 25.66%, 26%, 27.22%, 31.5%, 33.06%, 35.07% or 37.72%;
the total amount of hyaluronic acid and protamine is preferably 1.15%, 2.35%, 2.58%, 2.63%, 2.68%, 4.17% or 8.6%;
the particle size of the nanocomplex is preferably 20-95 nm, for example, 27.55±6.99 nm, 36.30±6.41 nm, 37.63±4.20 nm, 37.68±2.20 nm, 39.12±4.84 nm, 40.55±7.66 nm, 55.75±7.69 nm or 75.29 ± 14.53;
the Zeta potential of the nanocomplex is preferably -70 to -20 mV, for example, -63.7±2.66, -64.87±3.30, -52.07±2.15, -45.20±2.15, -44.87±0.45 mV, -43.20±2.75 mV, -40.23±6.92 or - 38.27 ± 13.10 mV.

4. The nanocomplex according to at least one of claims 1-3, wherein when the isoelectric point of the protein drug is 4-5.3 and the molecular weight is 200-255 kDa, the nanocomplex comprises 4-17% of protein drug, 0.05-0.25% of hyaluronic acid, 1-3.3% of protamine, 4-16.5% of "apolipoprotein and/or the mimetic peptide thereof", 42-53% of electrically neutral phospholipid and 31-38% of anionic phospholipid; wherein the total amount of the hyaluronic acid and the protamine is 1-3.5%;
the isoelectric point of the protein drug is preferably 4-4.8, for example 4.3;
the molecular weight of the protein drug is preferably 220-250 kDa, for example, 240 kDa;
the protein drug is preferably phycoerythrin;
the amount of the protein drug is preferably 4.8%, 8%, 8.5%, 11% or 16%;
the amount of the hyaluronic acid is preferably 0.08%, 0.13%, 0.15% or 0.2%;
the amount of the protamine is preferably 1%, 1.5% or 3%;
the amount of the "apolipoprotein and/or the mimetic peptide thereof' is preferably 4.12%, 5.8%, 10.35% or 16.37%;
the "apolipoprotein and/or the mimetic peptide thereof" is preferably ApoE;
the electrically neutral phospholipid is preferably phosphatidylcholine; the phosphatidylcholine is preferably DMPC;
the amount of the electrically neutral phospholipid is preferably 42.92%, 43.5%, 46.4%, 47.56% or 52.2%;
the anionic phospholipid is preferably DOPA;
the amount of the anionic phospholipid is preferably 31.08%, 31.5%, 33.6%, 34.44% or 37.8%;
the amount of the total amount of the hyaluronic acid and the protamine is preferably 1.08%, 1.15%, 1.2%, 1.63% or 3.2%;
the particle size of the nanocomplex is preferably 12-60 nm, for example, 20.30±5.89 nm, 23.79±7.91 nm, 27.31±10.84 nm, 28.96±8.74 nm, or 37.55±13.73 nm;
the Zeta potential of the nanocomplex is preferably -65 to -30 mV, for example, - 58.87±4.90, -56.33±3.26, -50.10±3.18, -43.93±14.03 or -36.83 ± 2.71 mV.

5. The nanocomplex according to at least one of claims 1-4, wherein when the isoelectric point of the protein drug is 9.3-11 and the molecular weight is 8-16 kDa, the nanocomplex comprises 31.5-34.5% of protein drug, 0.25-0.45% of hyaluronic acid, 0.1-0.35% of protamine, 3-5% of "apolipoprotein and/or the mimetic peptide thereof", 35-37.5% of electrically neutral phospholipid and 25-27.5% of anionic phospholipid; wherein the total amount of the hyaluronic acid and the protamine is 0.5-0.85%;
the isoelectric point of the protein drug is preferably 9.8-10.8, for example, 10.3;
the molecular weight of the protein drug is preferably 10-14 kDa, for example, 12.4 kDa;
the protein drug is preferably cytochrome C;
the amount of the protein drug is preferably 32-34%, for example, 33.07%;
the amount of the hyaluronic acid is preferably 0.3-0.5%, for example, 0.44%;
the amount of the protamine is preferably 0.1-0.3%, for example, 0.2%;
the amount of the "apolipoprotein and/or the mimetic peptide thereof' is preferably 3.5-4.5%, for example, 4.1%;
the "apolipoprotein and/or the mimetic peptide thereof" is preferably ApoE;
the electrically neutral phospholipid is preferably phosphatidylcholine; the phosphatidylcholine is preferably DMPC;
the amount of the electrically neutral phospholipid is preferably 35-37%, for example, 36.09%;
the anionic phospholipid is preferably DOPA;
the amount of the anionic phospholipid is preferably 25-27%, for example, 26.1%;
the total amount of the hyaluronic acid and the protamine is preferably 0.5-0.8%, for example, 0.64%;
the particle size of the nanocomplex is preferably 25-38 nm, for example, 31.11±3.44 nm;
the Zeta potential of the nanocomplex is preferably -25 to -15, for example, -21.03±2.47.

6. The nanocomplex according to at least one of claims 1-5, wherein when the isoelectric point of the protein drug is 7-9 and the molecular weight is 140-180 kDa, the nanocomplex comprises 5-43% of protein drug, 0.04-0.9% of hyaluronic acid, 1-14% of protamine, 2.5-20% of "apolipoprotein and/or the mimetic peptide thereof", 23.5-46% of electrically neutral phospholipid and 16-32% of anionic phospholipid; wherein the total amount of the hyaluronic acid and the protamine is 1-13.5%;
the isoelectric point of the protein drug is preferably 7.5-8.5, for example 8;
the molecular weight of the protein drug is preferably 150-170 kDa, for example, 160 kDa;
the protein drug is preferably an IgG antibody;
the amount of the protein drug is preferably 6%, 20%, 33% or 41%;
the amount of the hyaluronic acid is preferably 0.06%, 0.09%, 0.15%, 0.41% or 0.78%;
the amount of the protamine is preferably 1.3%, 1.7%, 2%, 4.3% or 13%;
the amount of the "apolipoprotein and/or the mimetic peptide thereof' is preferably 2.85%, 2.92%, 3.94%, 7.21%, 19.29%;
the "apolipoprotein and/or the mimetic peptide thereof" is preferably ApoE or a mimetic peptide of ApoA-I;
the electrically neutral phospholipid is preferably phosphatidylcholine; the phosphatidylcholine is preferably DMPC;
the amount of the electrically neutral phospholipid is preferably 24.36%, 34.22%, 41.21%, 42.34% or 45%;
the anionic phospholipid is preferably DOPA;
the amount of the anionic phospholipid is preferably 17.64%, 24.78%, 29.79%, 30% or 30.66%;
the total amount of the hyaluronic acid and the protamine is preferably 1.71%, 1.79%, 2.15%, 5.08% or 13.06%;
the particle size of the nanocomplex is preferably 15 to 95 nm, for example, 26.52±4.31 nm, 27.38±7.83 nm, 37.98±14.29 nm or 74.20 ± 14.23 nm;
the Zeta potential of the nanocomplex is preferably -70 to -20 mV, for example, - 57.33±2.31, -55.20±10.74, -43.87±9.68 or -31.57±4.67 mV.

7. The nanocomplex according to at least one of claims 1-6, wherein when the isoelectric point of the protein drug is 8.3-10.3 and the molecular weight is 15-40 kDa, the nanocomplex comprises 1-3% of protein drug, 0.2-0.6% of hyaluronic acid, 4.5-6.5% of protamine, 2.5-5% of "apolipoprotein and/or the mimetic peptide thereof", 54-57% of electrically neutral phospholipid and 32-35% of anionic phospholipid; wherein the total amount of the hyaluronic acid and the protamine is 5-7%;
the isoelectric point of the protein drug is preferably 8.8-9.8, for example, 9.3;
the molecular weight of the protein drug is preferably 20-33 kDa, for example, 26 kDa;
the protein drug is preferably a nerve growth factor β-NGF;
the amount of the protein drug is preferably 1.5-2.2%, for example, 1.78%;
the amount of the hyaluronic acid is preferably 0.3-0.5%, for example, 0.43%;
the amount of the protamine is preferably 5-6%, for example, 5.53%;
the amount of the "apolipoprotein and/or the mimetic peptide thereof" is preferably 3-4.5% for example, 3.82%;
the "apolipoprotein and/or the mimetic peptide thereof" is preferably ApoE;
the electrically neutral phospholipid is preferably phosphatidylcholine; the phosphatidylcholine is preferably DMPC;
the amount of the electrically neutral phospholipid is preferably 54.5-56%, for example, 55.32%;
the anionic phospholipid is preferably DOPA;
the amount of the anionic phospholipid is preferably 32.5-34%, for example, 33.12%;
the total amount of the hyaluronic acid and the protamine is preferably 5.5-6.5%, for example, 5.96%.

8. The nanocomplex according to at least one of claims 1-7, wherein when the isoelectric point of the protein drug is 6-8.5 and the molecular weight is 30-50 kDa, the nanocomplex comprises 15-20% of protein drug, 0.05-0.8% of hyaluronic acid, 0.8-1.6% of protamine, 3-6% of "apolipoprotein and/or the mimetic peptide thereof", 44-47% of electrically neutral phospholipid and 29-33% of anionic phospholipid; the total amount of the hyaluronic acid and the protamine is 0.3-2%;
the isoelectric point of the protein drug is preferably 6.5-8, for example 7.2;
the molecular weight of the protein drug is preferably 35-45 kDa, for example, 40 kDa;
the protein drug is preferably an active enzyme-based protein drug HRP;
the amount of the protein drug is preferably 16.5-19%, for example, 17.73%;
the amount of the hyaluronic acid is preferably 0.1-0.3%, for example, 0.14%;
the amount of the protamine is preferably 0.8-1.5%, for example, 0.83%;
the amount of the "apolipoprotein and/or the mimetic peptide thereof" is preferably 4-5%, for example, 4.58%;
the "apolipoprotein and/or the mimetic peptide thereof" is preferably ApoE;
the electrically neutral phospholipid is preferably phosphatidylcholine; the phosphatidylcholine is preferably DMPC;
the amount of the electrically neutral phospholipid is preferably 45-46%, for example, 45.61%;
the anionic phospholipid is preferably DOPA;
the amount of the anionic phospholipid is preferably 30.5-32%, for example, 31.11%;
the total amount of the hyaluronic acid and the protamine is preferably 0.5 to 1.5%, for example, 0.97%;
the particle size of the nanocomplex is preferably 20-57 nm, for example, 37.22±7.28 nm;
the Zeta potential of the nanocomplex is preferably -55 to -15, for example, -48.87±1.95;
when the isoelectric point of the protein drug is 4.4-6.4 and the molecular weight is 210-255 kDa, the nanocomplex comprises 10-33% of protein drug, 0.2-3% of hyaluronic acid, 3.5-7% of protamine, 3-6% of "apolipoprotein and/or the mimetic peptide thereof", 32-42% of electrically neutral phospholipid and 22-39% of anionic phospholipid; the total amount of the hyaluronic acid and the protamine is 4-8%;
the isoelectric point of the protein drug is preferably 4.9-5.9, for example, 5.4;
the molecular weight of the protein drug is preferably 220-250 kDa;
the protein drug is preferably catalase CAT;
the amount of the protein drug is preferably 11-32%, for example, 12.06 or 30.9%;
the amount of the hyaluronic acid is preferably 0.3-2.5%, for example, 0.5% or 1.53%;
the amount of the protamine is preferably 4-5.5%, for example, 4.85% or 5.1%;
the amount of the "apolipoprotein and/or the mimetic peptide thereof" is preferably 4-5%, for example, 4.43% or 4.5%;
the "apolipoprotein and/or the mimetic peptide thereof" is preferably ApoE;
the electrically neutral phospholipid is preferably phosphatidylcholine; the phosphatidylcholine is preferably DMPC;
the amount of the electrically neutral phospholipid is preferably 33-41%, for example, 34.2% or 39.91%;
the anionic phospholipid is preferably DOPA;
the amount of the anionic phospholipid is preferably 24-38%, for example, 24.8% or 37.22%;
the total amount of the hyaluronic acid and the protamine is preferably 5-7%, for example, 5.6% or 6.38%;
the particle size of the nanocomplex is preferably 55-70 nm, for example, 63.62±1.97 nm;
the Zeta potential of the nanocomplex is preferably -25 to -15 mV, for example, - 19.43±1.96 mV.

9. A preparation method for the nanocomplex according to at least one of claims 1-8, which is prepared by the following method I or method II:
method I:
S1. mixing the protein drug, the hyaluronic acid and the protamine to form a nanogel;
S2. preparing a liposome using the lipid component by conventional methods;
S3. co-incubating the nanogel with the liposome to form a nanogel-loading liposome;
S4. self-assembling the nanogel-loading liposome and the "apolipoprotein and/or the mimetic peptide thereof" to form the nanocomplex;
method II:
S1. mixing the protein drug, the hyaluronic acid and the protamine to form a nanogel;
S2. preparing a liposome using the lipid component by conventional methods;
S3. preparing a nanogel-loading liposome by using a microfluidic chip to combine the nanogel with the liposome, and obtaining a liposome loaded with protein drug after removing the solvent by ultrafiltration;
S4. self-assembling the liposome loaded with protein drug and the "apolipoprotein and/or the mimetic peptide thereof" to form the nanocomplex.

10. A use of the nanocomplex according to at least one of claims 1-8 in the delivery of the protein drug; preferably, the nanocomplex is used for intracellular, in vivo or intracerebral delivery of the protein drug.
